(19) Europäisches Patentamt / European Patent Office / Office européen des brevets

(11) **EP 2 596 113 B1**

(12) **EUROPEAN PATENT SPECIFICATION**

(45) Date of publication and mention of the grant of the patent:
**12.09.2018 Bulletin 2018/37**

(21) Application number: **11734090.1**

(22) Date of filing: **19.07.2011**

(51) Int Cl.:
*C12P 19/14* (2006.01)     *C07H 3/04* (2006.01)
*C07H 3/06* (2006.01)

(86) International application number:
**PCT/EP2011/062355**

(87) International publication number:
**WO 2012/010597 (26.01.2012 Gazette 2012/04)**

(54) **GALACTO-OLIGOSACCHARIDE-CONTAINING COMPOSITION AND A METHOD OF PRODUCING IT**

GALACTOOLIGOSACCHARID-ENTHALTENDE ZUSAMMENSETZUNG UND IHR HERSTELLUNGSVERFAHREN

COMPOSITION CONTENANT DES GALACTO-OLIGOSACCHARIDES ET SON PROCÉDÉ DE PRODUCTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **19.07.2010 US 365560 P**
**19.07.2010 EP 10169981**

(43) Date of publication of application:
**29.05.2013 Bulletin 2013/22**

(73) Proprietor: **DuPont Nutrition Biosciences ApS**
**1411 Copenhagen K (DK)**

(72) Inventors:
• **BERTELSEN, Hans**
**DK-6920 Videbæk (DK)**
• **LANGBORG WEJSE, Peter**
**DK-8200 Aarhus N (DK)**

(74) Representative: **D Young & Co LLP**
**120 Holborn**
**London EC1N 2DY (GB)**

(56) References cited:
**WO-A2-01/90317**

• **SCHRODER S ET AL: "Synthesis of oligosaccharides as potential novel food components and upscaled enzymatic reaction employing the beta-galactosidase from bovine testes", TETRAHEDRON, ELSEVIER SCIENCE PUBLISHERS, AMSTERDAM, NL, vol. 60, no. 11, 8 March 2004 (2004-03-08), pages 2601-2608, XP004492591, ISSN: 0040-4020, DOI: DOI:10.1016/J.TET.2004.01.029**
• **MURATA T ET AL: "ENZYMATIC SYNTHESIS OF SULFATED DISACCHARIDES USING BETA-D-GALACTOSIDASE-CATALYZED TRANSGLYCOSYLATION", BIOSCIENCE BIOTECHNOLOGY BIOCHEMISTRY, JAPAN SOCIETY FOR BIOSCIENCE, BIOTECHNOLOGY, AND AGROCHEMISTRY, TOKYO, JAPAN, vol. 65, no. 11, 1 January 2001 (2001-01-01), pages 2456-2464, XP009062212, ISSN: 0916-8451, DOI: DOI:10.1271/BBB.65.2456**
• **ZARATE S ET AL: "OLIGOSACCHARIDE FORMATION DURING ENZYMATIC LACTOSE HYDROLYSIS: A LITERATURE REVIEW", JOURNAL OF FOOD PROTECTION, INTERNATIONAL ASSOCIATION FOR FOOD PROTECTION, US, vol. 53, 1 March 1990 (1990-03-01), pages 262-268, XP009076545, ISSN: 0362-028X**

- GOSLING A ET AL: "Recent advances refining galactooligosaccharide production from lactose", FOOD CHEMISTRY 20100715 ELSEVIER LTD GBR LNKD-DOI:10.1016/J.FOODCHEM.2009.12.063, vol. 121, no. 2, 15 July 2010 (2010-07-15), pages 307-318, XP002612677, ISSN: 0308-8146
- HUBER R E ET AL: "A quantitation of the factors which affect the hydrolase and transgalactosylase activities of [beta] galactosidase (E. coli) on lactose", BIOCHEMISTRY 1976, vol. 15, no. 9, 1976, pages 1994-2001, XP002612678, ISSN: 0006-2960
- RABIU BODUN A ET AL: "Synthesis and fermentation properties of novel galacto-oligosaccharides by beta-galactosidases from Bifidobacterium species", APPLIED AND ENVIRONMENTAL MICROBIOLOGY, vol. 67, no. 6, June 2001 (2001-06), pages 2526-2530, XP002613005, ISSN: 0099-2240
- DUMORTIER V ET AL: "PURIFICATION AND PROPERTIES OF A BETA-D-GALACTOSIDASE FROM BIFIDOBACTERIUM BIFIDUM EXHIBITING A TRANSGALACTOSYLATION REACTION", BIOTECHNOLOGY AND APPLIED BIOCHEMISTRY, ACADEMIC PRESS, US, vol. 19, no. 19, 1 January 1994 (1994-01-01), pages 341-354, XP002902258, ISSN: 0885-4513
- THEODOROS K GOULAS ET AL: "Molecular cloning and comparative analysis of four [beta]-galactosidase genes from Bifidobacterium bifidum NCIMB41171", APPLIED MICROBIOLOGY AND BIOTECHNOLOGY, SPRINGER, BERLIN, DE, vol. 76, no. 6, 8 August 2007 (2007-08-08), pages 1365-1372, XP019538760, ISSN: 1432-0614, DOI: DOI:10.1007/S00253-007-1099-1
- TZORTZIS GEORGE ET AL: "A novel galactooligosaccharide mixture increases the bifidobacterial population numbers in a continuous in vitro fermentation system and in the proximal colonic contents of pigs in vivo.", THE JOURNAL OF NUTRITION JUL 2005 LNKD-PUBMED:15987856, vol. 135, no. 7, July 2005 (2005-07), pages 1726-1731, XP002612999, ISSN: 0022-3166
- KOBATA AKIRA: "Structures and application of oligosaccharides in human milk", PROCEEDINGS OF THE JAPAN ACADEMY SERIES B PHYSICAL AND BIOLOGICAL SCIENCES, vol. 86, no. 7, July 2010 (2010-07), pages 731-747, XP002612998, ISSN: 0386-2208

Remarks:
The file contains technical information submitted after the application was filed and not included in this specification

**Description**

**FIELD OF THE INVENTION**

The present invention relates to a galacto-oligosaccharide-containing composition as well as an efficient method of producing it.

**BACKGROUND**

Human breast milk is known to contain a number of different oligosaccharides which are ascribed some of the beneficial health effects of breast feeding infants (Kunz *et al.* (2000)). For example, some oligosaccharides, such as FOS, GOS, or inulin, are so-called prebiotics, which means that they promote the beneficial bacteria of the gastrointestinal system and disfavour the harmful bacteria. Oligosaccharides are, due to their health promoting effects, frequently used in functional food products, such as infant formulas and clinical nutrition.

There are several approaches to the production of oligosaccharides. One approach is based on isolating oligosaccharides from naturally occurring sources. Fructose-oligosaccharide (FOS) and inulin are for example found naturally in Jerusalem artichoke, burdock, chicory, leeks, onions and asparagus and may be isolated from these crops. Preparation of inulin from chicory roots is e.g. described in Frank (2002). This approach to the production of oligosaccharides is limited by the availability of suitable crops and may be impossible to implement for more complex oligosaccharides.

Another approach is based on enzymatic synthesis in which enzymes catalyse the synthesis of the oligosaccharides. Yun (1996) describes the enzymatic production of fructo-oligosaccharides using enzymes having fructosyltransferase activity and using sucrose as substrate for the enzyme. Dumortier et al, Biotechnol Appl. Biochem, 1994, Vol. 19, pp. 341-354, discloses a method of producing a composition comprising a galacto-oligosaccharide, and the use of cells comprising a β-D-galactosidase. The purified β-D-galactosidase has a specific galactosyltransferase activity at pH 4.8 and at temperature of 45°C. The enzyme releases the leaving group of the galactosyl donor, e.g. lactose, and transfers the galactosyl group of the galactosyl donor to the galactosyl acceptor, thus forming the galacto-oligosaccharide in an amount of up to 29% of the total amount of sugar. The transfer reaction of the galactosyl group happens when using lactose having a concentration of 0.5 M as donor and the concentration of acceptors ranging from 0.75 M to 1.5 M, the molar ratio between the galactosyl donors and the galactosyl acceptors being 1:1.5-1:3. The T-value is about 0.78. It is also shown that many interesting sugars, such as mannose, fucose and fructose, cannot be used as galactosyl acceptors by use of the disclosed enzyme. Another example of enzymatic synthesis is described in WO 01/90,317 A2 which discloses a method of producing galacto-oligosaccharides (GOS) of the formula Gal-Gal-Glc using a special beta-galactosidase enzyme and lactose as substrate. GenBank, accession number AJ224435.2, discloses an updated version (from 5 August 2003) of the sequence of the beta-galactosidase enzyme disclosed in WO 01/90317.

**SUMMARY OF THE INVENTION**

An object of the invention is to provide improved methods of producing galacto-oligosaccharides. It is furthermore an object of the invention to provide improved compositions containing galacto-oligosaccharides.

The present inventors have observed that, surprisingly, enzymes having beta-galactosidase activity, and preferably having a T-value of at most 0.6, can be used for highly effective synthesis of a special type of galacto-oligosaccharides, in which the galactosyl acceptor is different from the galactosyl donor.

Thus, an aspect of the invention relates to a method of producing a composition comprising one or more galacto-oligosaccharides, the method comprising the steps of:

    a) providing a mixture comprising

- a galactosyl donor comprising a galactosyl group bound to a leaving group, which galactosyl donor has a molar weight of at most 350 g/mol,
- a galactosyl acceptor which is different from the galactosyl donor, said galactosyl acceptor is a saccharide or a sugar-alcohol, and

    wherein the molar ratio between the galactosyl acceptor and the galactosyl donor is at least 1:10, and wherein the mixture comprises at least 0.05 mol/L of the galactosyl acceptor and the galactosyl donor in a concentration of at most 0.7 mol/L,

    b) providing an enzyme having beta-galactosidase activity and having a T-value of at most 0.6, said enzyme contacting the mixture, the determination of the T-value of a beta-galactosidase enzyme is performed by providing a solution consisting of the beta-galactosidase enzyme in an amount sufficient to use 33% (w/w) of added lactose in 1 hour at 37°C for four hours and calculated using the formula:

$$\text{T-value} = \frac{\text{amount of produced galactose (in mol)}}{\text{amount of used lactose (in mol)}}$$

and

c) allowing the enzyme to release the leaving group of the galactosyl donor and transfer the galactosyl group of the galactosyl donor to the galactosyl acceptor, thus forming the galacto-oligosaccharide, and thereby obtaining the composition comprising the one or more galacto-oligosaccharide(s).

This invention opens up for cheap and efficient production of complex galacto-oligosaccharide compositions in high yield. The present invention furthermore appears to reduce the degree of self-galactosylation of the galactosyl donor, which may result in undesired by-products, which are expensive to remove from the composition.

Preferably, the enzyme has transgalactosylation activity in addition to beta-galactosidase activity. It may also be preferred that the enzyme has a T-value of at most 0.5.

In the context of the present invention the term "transgalactosylation activity" of a beta-galactosidase enzyme relates to the ability of the enzyme to transfer a galactosyl group from a donor molecule, e.g. a lactose molecule, to a non-water molecule, e.g. another lactose molecule.

The T-value is a measure of the transgalactosylation efficiency of a beta-galactosidase enzyme using lactose both as galactosyl donor and acceptor. The determination of the T-value of a beta-galactosidase enzyme is performed according to the assay and the formula described in Example 2. The T-value is calculated using the formula:

$$\text{T-value} = \frac{\text{amount of produced galactose (in mol)}}{\text{amount of used lactose (in mol)}}$$

A lactase enzyme without any transgalactosylation activity will produce one mol galactose for each used mol lactose and would have a T-value of 1. A beta-galactosidase having an extremely high transgalactosylation activity would use nearly all the galactosyl groups from the lactose for transgalactosylation instead of generating galactose, and would consequently have a T-value near 0.

A composition comprising one or more galacto-oligosaccharide(s) is obtainable by the method as described herein.

Additional objects and advantages of the invention are described below.

**BRIEF DESCRIPTION OF THE FIGURES**

Figure 1a shows a HPLC chromatogram of the mixture described in Example 3 (containing lactose and L-fucose) before incubation with the enzyme.

Figure 1b shows a HPLC chromatogram of the mixture of Example 3 after incubation with the enzyme, where peaks of L-fucose-containing galacto-oligosaccharides (peaks 6, 7 and 8) are clearly present.

Figure 2 contains a plot of the concentration (arbitrary units) of lactose, glucose and galactose of the mixture of Example 3 during the enzymatic reaction.

Figure 3 contains a plot of the concentration (arbitrary units) of the oligosaccharides Gal-Fuc, Gal-Gal-Fuc/Gal-Gal-Glc, and Gal-Gal-Gal-Fuc/Gal-Gal-Gal-Glc of the mixture of Example 3 during the enzymatic reaction.

Figure 4 contains a plot of the concentration (arbitrary units) of the oligosaccharides Gal-Fuc, Gal-Gal-Fuc/Gal-Gal-Glc, and Gal-Gal-Gal-Fuc/Gal-Gal-Gal-Glc of the mixture of Example 4 during the enzymatic reaction.

Figure 5 contains a plot of the concentration (arbitrary units) of the oligosaccharides Gal-GalNAc, Gal-Gal- Gal-

NAc/Gal-Gal-Glc, and Gal-Gal-Gal-GalNAc /Gal-Gal-Gal-Glc of the mixture of Example 5 during the enzymatic reaction.

Figure 6 contains a plot of the concentration (arbitrary units) of the oligosaccharides Gal-Xyl, Gal-Gal- Xyl/Gal-Gal-Glc, and Gal-Gal-Gal- Xyl/Gal-Gal-Gal-Glc of the mixture of Example 6 during the enzymatic reaction.

## DETAILED DESCRIPTION OF THE INVENTION

As mentioned, an aspect of the invention relates to a method of producing a composition comprising one or more galacto-oligosaccharide(s), the method comprising the steps of:

a) providing a mixture comprising

- a galactosyl donor comprising a galactosyl group bound to a leaving group, which galactosyl donor has a molar weight of at most 350 g/mol,
- a galactosyl acceptor which is different from the galactosyl donor, said galactosyl acceptor is a saccharide or a sugar-alcohol, and

wherein the molar ratio between the galactosyl acceptor and the galactosyl donor is at least 1:10, and wherein the mixture comprises at least 0.05 mol/L of the galactosyl acceptor and the galactosyl donor in a concentration of at most 0.7 mol/L,

b) providing an enzyme having beta-galactosidase activity and preferably having a T-value of at most 0.6, said enzyme contacting the mixture, the determination of the T-value of a beta-galactosidase enzyme is performed according to the assay in Example 2 and calculated using the formula:

$$\text{T-value} = \frac{\text{amount of produced galactose (in mol)}}{\text{amount of used lactose (in mol)}}$$

and

c) allowing the enzyme to release the leaving group of the galactosyl donor and transfer the galactosyl group of the galactosyl donor to the galactosyl acceptor, thus forming the galacto-oligosaccharide, and thereby obtaining the composition comprising the one or more galacto-oligosaccharide(s).

In the context of the present invention, the term "glycosyl group" relates to a group obtained by removing one or two hydroxyl groups from a monosaccharide or a lower oligosaccharide, such as a di- or tri-saccharide, or from corresponding sugar-alcohols. The term is used herein to describe various building blocks of galactosyl donors, galactosyl acceptors and oligosaccharides.

The abbreviations of the most common saccharides and their corresponding glycosyl groups are shown below.

| Saccharide | Abbreviation | Name of glycosyl group |
|---|---|---|
| glucose | Glc | glucosyl |
| galactose | Gal | galactosyl |
| fucose | Fuc | fucosyl |
| mannose | Man | mannosyl |
| xylose | Xyl | xylosyl |
| N-acetylgalactosamine | GalNAc | N-acetylgalactosaminyl |
| Lactose | Lac | lactosyl |

In the context of the present invention, the term "oligosaccharide" relates to a molecule comprising at least two glycosyl groups, and preferably at least three, which may be different or the same type. The at least two glycosyl groups are preferably bound via an O-glycosylic bond. An oligosaccharide may be a linear chain of glycosyl groups or it may have a branched structure. Oligosaccharides may e.g. be represented as a stoichiometric formula, e.g. $(Gal)_3Glc$, or as general formulas, e.g. Gal-Gal-Gal-Glc, Gal-Gal-Glc-Gal, or Gal-(Gal-)Glc-Gal. The stoichiometric formulas provide information regarding which glycosyl groups an oligosaccharide, or a group of oligosaccharides, contains, but not the relative position of these, whereas the general formulas also contain general information regarding the relative positions of the glycosyl groups.

In the context of the present invention the term "homo-oligosaccharide" relates to an oligosaccharide containing only one type of glycosyl group. Examples of homo-oligosaccharides are Gal-Gal-Gal-Gal and Glc-Glc-Glc.

In the context of the present invention the term "hetero-oligosaccharide" relates to an oligosaccharide which contains different glycosyl groups, e.g. Gal-Gal-Glc, or Gal-Gal-Fuc.

In the context of the present invention, the prefix "galacto-" used together with the term "oligosaccharide" indicates that the oligosaccharide contains galactosyl groups as the repeating unit. The "homo-" or "hetero-" prefix may be used together with the "galacto-" prefix. Both Gal-Gal-Glc and Gal-Gal-Gal-Gal are galacto-oligosaccharides. Gal-Gal-Glc is a hetero-galacto-oligosaccharide and Gal-Gal-Gal-Gal is a homo-galacto-oligosaccharide.

In the context of the present invention, "X" represents a galactosyl acceptor as defined herein. "-X" represents the glycosyl group corresponding to the galactosyl acceptor, and particularly the glycosyl group bound to another group. "-" symbolises the bond. The glycosyl group is preferably bound via the 3-, 4-, 5- or 6-position of the glycosyl group, and preferably via an O-glycosylic bond.

In the context of the present invention, "Gal-" represents a galactosyl group bound to another group, preferably via the 1-position of the galactosyl group, and preferably via an O-glycosylic bond.

In the context of the present invention, "-Gal-" represents a galactosyl group bound to two other groups. The left bond is preferably made via the 4- or 6-position of the galactosyl group, and preferably via an O-glycosylic bond. The right bond is preferably made via the 1-position of the galactosyl group, and preferably via an O-glycosylic bond.

Bonds between two galactosyl groups are typically 1-4 or 1-6 bonds, and normally O-glycosylic bonds. A bond between a galactosyl group and a nitrogen-containing acceptor may alternatively be an N-glycosylic bond.

In the context of the present invention the terms "method" and "process" are used interchangeably.

Step a) involves the provision of the mixture in which the oligosaccharides are to be produced.

The mixture is preferably a liquid mixture and may e.g. be an aqueous solution containing the galactosyl acceptor and the galactosyl donor.

In some embodiments of the invention the molar ratio between the galactosyl acceptor and the galactosyl donor of the mixture of step a) is at least 1:5, preferably at least 1:1, and even more preferably at least 5:1. For example, the molar ratio between the galactosyl acceptor and the galactosyl donor of the mixture of step a) may be at least 10:1, such as at least 15:1.

The molar ratio between the galactosyl acceptor and the galactosyl donor of the mixture of step a) may e.g. be in the range of 1:10-100:1.

In some embodiments of the invention the molar ratio between the galactosyl acceptor and the galactosyl donor of the mixture of step a) is in the range of 1:10-50:1, preferably in the range of 1:5-30:1, and even more preferably in the range of 1:1-20:1. For example, the molar ratio between the galactosyl acceptor and the galactosyl donor of the mixture of step a) may e.g. be in the range of 2:1-40:1, preferably in the range of 4:1-30:1, and even more preferably in the range of 10:1-25:1.

As mentioned, the galactosyl donors contain a galactosyl group covalently bound to a leaving group. The galactosyl group is preferably a $\beta$-D-galactopyranosyl group. Furthermore, the galactosyl group is preferably bound to the leaving group via an O-glycosidic bond from the 1-position of the galactosyl group.

The leaving group of the galactosyl donor may for example be a glycosyl group and/or a sugar-alcohol group. If the leaving group is a glycosyl group of a mono- or disaccharide or a corresponding sugar-alcohol, the galactosyl group is preferably bound to the leaving group via an O-glycosidic bond from the 1-position of the galactosyl group, which bond attaches to the 4-position of a monosaccharide-type leaving group or to the 4'-position of a disaccharide-type leaving group.

In the context of the present invention, the phrase "Y and/or X" means "Y" or "X" or "Y and X". Along the same line of logic, the phrase "$X_1$, $X_2$,..., $X_{i-1}$, and/or $X_i$" means "$X_1$" or "$X_2$" or... or "$X_{i-1}$" or "$X_i$" or any combination of the components : $_X 1$, $X_2$,...$X_{i-1}$, and $X_i$.

According to the invention the galactosyl donor has a molar weight of at most 350 g/mol.

Disaccharides are a presently preferred type of galactosyl donor. Alternatively, or additionally, tri-saccharides may be used as galactosyl donors as well. Thus, it is envisioned that the mixture may contain a combination of different galactosyl donors.

In some preferred embodiments of the invention the galactosyl donor is lactose. Another example of a useful galactosyl donor is lactulose. Yet an example of a useful galactosyl donor is lactitol.

In the context of the present invention the term "lactose" relates to the disaccharide $\beta$-D-galactopyranosyl-(1→4)-D-glucose, which is also referred to as milk sugar, and which is the most predominant saccharide of bovine milk.

The galactosyl donor may be provided via any useful galactosyl donor source, both industrially refined sources, such as purified lactose, and/or natural sources, such as whey permeate, i.e. deproteinated whey prepared by ultrafiltration of whey.

The galactosyl acceptor is a saccharide or a sugar-alcohol and capable of accepting a galactosyl group from the enzyme and typically contains hydroxyl groups, and preferably alcoholic hydroxyl groups. The term "accepting" means that the galactosyl group of the donor should be covalently bound to the acceptor, e.g. via an O-glycosylic bond.

In some embodiments of the invention the galactosyl acceptor comprises one or more alcoholic hydroxyl group(s). For example, the galactosyl acceptor may be a polyol.

In the context of the present invention the term "polyol" relates to a molecule comprising at least two alcoholic hydroxyl groups.

In some preferred embodiments of the invention the galactosyl acceptor is not lactose. It may furthermore be preferred that the galactosyl acceptor is not glucose.

In some preferred embodiments of the invention the galactosyl acceptor is different from the galactosyl donor. It is particularly preferred to use a relatively cheap galactosyl donor, such as lactose, as galactosyl source and a biologically interesting acceptor, such as fucose, as galactosyl acceptor.

In some embodiments of the invention the galactosyl acceptor is not lactose, galactose, or glucose.

In some embodiments of the invention the galactosyl acceptor is not glucose or oligosaccharides of the general formula Gal-(Gal)$_i$-Glc, where i is a non-negative integer, i.e. for example 0, 1, 2, 3, or 4.

In some embodiments of the invention the galactosyl acceptor is not galactose or oligosaccharides of the general formula Gal-(Gal)$_i$-Gal, where i is a non-negative integer.

Galactosyl acceptors having various molar weights may be used, but galactosyl acceptors having a molar weight of at least 100 g/mol are presently preferred.

In some embodiments of the invention the galactosyl acceptor has a molar weight of at most 1000 g/mol. For example, the galactosyl acceptor may have a molar weight of at most 500 g/mol. It may even be preferred that the galactosyl acceptor has a molar weight of at most 350 g/mol. The galactosyl acceptor may for example have a molar weight of at most 200 g/mol.

In some preferred embodiments of the invention the galactosyl acceptor is a saccharide. The galactosyl acceptor may for example be a mono-saccharide. Alternatively, the galactosyl acceptor may be a di-saccharide.

For example, the galactosyl acceptor may be a pentose. The galactosyl acceptor may e.g. be arabinose. Another example of a useful pentose is xylose. Yet an example of a useful pentose is ribose. The galactosyl acceptor may for example be a pentose selected from the group consisting of arabinose, xylose, and ribose.

Hexoses are another group of useful galactosyl acceptors. The galactosyl acceptor may e.g. be mannose. Another example of a useful hexose is galactose. Yet an example of a useful hexose is tagatose. A further example of a useful hexose is fructose. The galactosyl acceptor may for example be a hexose selected from the group consisting of mannose, galactose, tagatose, and fructose.

In some preferred embodiments of the invention the galactosyl acceptor is a deoxy-hexose. The galactosyl acceptor may for example be fucose, such as e.g. D-fucose, L-fucose, or a mixture thereof.

Alternatively, the galactosyl acceptor may be an oligosaccharide, such as e.g. a di-saccharide or a tri-saccharide. An example of a useful di-saccharide is maltose. Another example of a useful di-saccharide is lactulose.

Yet a useful group of galactosyl acceptors is saccharide derivatives.

In the context of the present invention the term "saccharide derivative" pertains to a saccharide containing one or more non-hydroxyl functional group(s). Examples of such functional groups are a carboxyl group, an amino group, an N-acetylamino group and/or a thiol group. Saccharides which contain an aldehyde group at the 1-position or a ketone group at the 2-position are not considered saccharide derivatives as such unless the saccharides comprise some of the non-hydroxyl functional groups mentioned above.

An example of a useful saccharide derivative is N-acetyl galactosamine. Another example of a useful saccharide derivative is sialic acid. Yet an example of a useful saccharide derivative is sialyl lactose. Thus, the galactosyl acceptor may be a saccharide derivative selected from the group consisting of N-acetyl galactosamine, sialic acid, and sialyl lactose.

Another group of useful galactosyl acceptors is sugar alcohols. Thus, in some embodiments of the invention the galactosyl acceptor is a sugar alcohol. Examples of useful sugar alcohols are sorbitol, xylitol, lactitol, and/or maltitol.

Contrary to the above-mentioned galactosyl acceptors, the present inventors have found that N-acetyl glucosamine and glucose are less efficient galactosyl acceptors. Thus, in some embodiments of the invention the galactosyl acceptor is not glucose or N-acetyl glucosamine.

The mixture may contain one or more further galactosyl acceptor(s) different from the first type of galactosyl acceptor. The different types of galactosyl acceptors of the mixture may e.g. be selected among the galactosyl acceptor types mentioned herein.

In some preferred embodiments of the invention the produced galactosylated acceptors act as a new type of galactosyl acceptor and can be galactosylated as well. In this way, galacto-oligosaccharides may be produced which have the stoichiometric formula Gal$_{i+1}$X, where i is a non-negative integer. Normally, the most predominant species are

GalX, Gal$_2$X, and Gal$_3$X.

In other preferred embodiments of the invention the produced galactosylated acceptors act as a new type of galactosyl acceptor and can be galactosylated as well. In this way, galacto-oligosaccharides may be produced which have the general formula Gal-(Gal)$_i$-X, where i is a non-negative integer. Normally, the most predominant species are Gal-X, Gal-Gal-X, and Gal-Gal-Gal-X.

In some embodiments of the invention the mixture of step a) comprises the galactosyl donor in a concentration of at most 0.7 mol/L, preferably at most 0.4 mol/L, and even more preferably at most 0.2 mol/L. The mixture may e.g. comprise the galactosyl donor in a concentration in the range of 0.001-0.7 mol/L, preferably in the range of 0.01-0.5 mol/L, and even more preferred in the range of 0.02-0.2 mol/L.

Alternatively, the mixture of step a) may comprise the galactosyl donor in a concentration of at most 0.3 mol/L, preferably at most 0.1 mol/L, and even more preferably at most 0.05 mol/L. The mixture may e.g. comprise the galactosyl donor in a concentration in the range of 0.001-0.2 mol/L, preferably in the range of 0.005-0.1 mol/L, and even more preferred in the range of 0.01-0.05 mol/L.

It should be noted that galactosylated galactosyl acceptor and galactosylated galactosyl donor may to a limited extent act as a galactosyl donor, but galactosylated galactosyl acceptor and galactosylated galactosyl donor are not considered a galactosyl donor in the context of the present invention and do not contribute to the concentrations or ratios of galactosyl donor mentioned herein.

The galactosyl acceptor may be used in a range of difference concentrations. It is, however, preferred to avoid saturating the mixture with the galactosyl acceptor since excess galactosyl acceptor normally has to be removed from the galacto-oligosaccharide-containing composition of the invention.

In some embodiments of the invention the mixture of step a) comprises the galactosyl acceptor in an amount of at least 0.05 mol/L, preferably at least 0.10 mol/L, and even more preferably at least 0.30 mol/L. Even higher concentrations of the galactosyl acceptor may be preferred, thus the mixture of step a) may e.g. comprise the galactosyl acceptor in an amount of at least 0.5 mol/L, preferably at least 0.7 mol/L, and even more preferably at least 1 mol/L.

The mixture may e.g. comprise the galactosyl acceptor in a concentration in the range of 0.05 mol/L - 5 mol/L, preferably in the range of 0.1 mol/L - 2 mol/L, and even more preferably in the range of 0.3 mol/L - 1 mol/L.

However, in some embodiments a relatively low concentration of the galactosyl acceptor is preferred in which case the mixture may e.g. comprise the galactosyl acceptor in a concentration of at most 2 mol/L, preferably at most 0.5 mol/L, and even more preferably at most 0.2 mol/L. For example, the mixture may comprise the galactosyl acceptor in a concentration in the range of 0.05 mol/L - 2 mol/L, preferably in the range of 0.06 mol/L - 1 mol/L, and even more preferably in the range of 0.08 mol/L - 0.8 mol/L.

In addition to galactosyl acceptor and galactosyl donor, the mixture may furthermore contain various additives for optimizing the conditions for the enzymatic reaction.

The mixture may for example contain one or more pH buffer(s) for adjusting the pH of the mixture to the pH optimum of the enzyme. Alternatively, or in addition, the mixture may comprise water soluble salts containing one or more metal ions. Depending on the specific enzyme, metal ions such as $Ca^{2+}$, $Zn^{2+}$, or $Mg^{2+}$ may e.g. be used. Note, however, that some enzymes are insensitive to the presence of metal ions in the mixture.

Conventional methods of synthesising oligosaccharides often employ water-activity-lowering agents, such as e.g. glycerol, ethylene glycol, propylene glycol, polyethyleneglycol (PEG). The present invention advantageously makes it possible to perform efficient synthesis of galacto-oligosaccharides without the use of such water-activity-lowering agents. Thus, in some preferred embodiments of the invention the mixture contains water-activity-lowering agent in an amount of at most 5% by weight relative to the weight of the mixture, preferably at most 1 % by weight, and even more preferably at most 0.1% by weight. For example, the mixture may contain water-activity-lowering agent in an amount of at most 0.05% by weight relative to the weight of the mixture.

The mixture of step a) or the ingredients forming the mixture may have been heat treated before the reaction with enzyme to avoid microbial growth during the reaction. The usual heat treatment processes, such as pasteurisation (e.g. 72 degrees C for 15 seconds), high pasteurisation (e.g. 90 degrees C for 15 seconds), or UHT treatment (e.g. 140 degrees C for 4 seconds), may be used. Care should be taken when heat treating temperature labile enzymes.

Step b) involves the provision of an enzyme, which has beta-galactosidase activity with a T-value of at most 0.6. It should be noted that the method may furthermore involve the use of additional enzymes, e.g. enzymes having a different enzymatic activity than beta-galactosidase activity or transgalactosylation activity.

In the context of the present invention the term "beta-galactosidase activity" relates to enzymatic catalysis of the hydrolysis of terminal non-reducing β-D-galactose residues in β-D-galactosides, such as lactose. The enzyme used in the invention preferably belongs to the class EC 3.2.1.23.

In some embodiments of the invention, the T-value of the enzyme is at most 0.5. Preferably the T-value of the enzyme may be at most 0.4. It may even be more preferred that the T-value of the enzyme is at most 0.3.

Even lower T-values may be preferred, such as at most 0.2.

Useful enzymes may e.g. be derived from a peptide (a β-galactosidase) prepared as described in Example 1.

In some embodiments of the invention the enzyme may contain one or more glycosylated amino acid(s). Alternatively, or in addition, the enzyme may contain one or more phosphorylated amino acid(s). Alternatively, none of the amino acids of the enzyme are glycosylated or phosphorylated.

In some preferred embodiments of the invention the enzyme comprises at least two sub-units, each sub-unit consisting of an enzyme as defined above.

The enzyme preferably contacts the mixture and is thereby brought into contact with both the galactosyl acceptor and the galactosyl donor.

In some embodiments of the invention the mixture comprises the enzyme. The enzyme may e.g. be present in the mixture in dissolved form, e.g. as single enzyme molecules or as soluble aggregate of enzyme molecules.

In other embodiments of the invention the enzyme is separate from the mixture, but brought in contact with the galactosyl acceptor and the galactosyl donor by contacting the enzyme with the mixture. For example, enzyme immobilised on a stationary solid phase may be used. Examples of useful stationary solid phases are e.g. a filter, a packed bed of enzyme-containing particles, or similar structures.

Alternatively, the solid phase may e.g. be a free flowing, particulate solid phase, e.g. organic or inorganic beads, forming part of the mixture.

Details relating to the industrial use of enzymes including immobilisation techniques and suitable solid phase types can be found in Buchholz (2005).

The enzyme is preferably used in a sufficient activity to obtain an acceptable yield of galacto-oligosaccharides. The optimal activity depends on the specific implementation of the process and can easily be determined by the person skilled in the art.

If a high turn-over of galactosyl donor and a high yield of galacto-oligosaccharide is required, it may be preferred to use the enzyme in a relatively high activity. For example, the activity of the enzyme may be such that the turn-over of the galactosyl donor is at least 0.02 mol/(L*h), preferably at least 0.2 mol/(L*h), and even more preferably at least 2 mol/(L*h).

The enzymatic reaction takes place during step c). As soon as the mixture is exposed to the right conditions, which may be almost immediately when the galactosyl acceptor and the galactosyl donor are brought in contact with the enzyme, the transgalactosylation usually starts, and in some embodiments of the invention steps b) and c) occur simultaneously.

The enzyme is capable of releasing the leaving group of the galactosyl donor and transferring the galactosyl group of the galactosyl donor to the galactosyl acceptor. For example, if the galactosyl donor is lactose, glucose is released and the galactosyl group is transferred to the acceptor. The enzyme acts as catalyst during the enzymatic reaction.

In some preferred embodiments of the invention the enzyme furthermore transfers galactosyl groups to already galactosylated galactosyl acceptors, thereby generating galactosyl acceptors containing two, three or even more galactosyl groups.

The pH of the mixture is preferably near the optimum pH of the enzyme. In some embodiments of the invention the pH of the mixture during step c) is in the range of pH 3-9. For example, the pH of the mixture during step c) may be in the range of pH 4-8, such as in the range of pH 5-7.5.

Similar to the pH, the temperature of the mixture is preferably adjusted to the optimum temperature of the used enzyme. In some embodiments of the invention the temperature during step c) is in the range of 10-80 degrees C. The temperature during step c) may e.g. be in the range of 20-70 degrees C, preferably in the range of 25-60 degrees C, and even more preferably in the range of 30-50 degrees C.

In the context of the present invention, the term "optimum pH of the enzyme" relates to the pH where the enzyme has the highest transgalactosylation activity. Along the same lines, the term "optimum temperature of the enzyme" relates to the temperature where the enzyme has the highest transgalactosylation activity.

The inventors have discovered that the present method surprisingly provides a high yield of galacto-oligosaccharides even though a relatively low concentration of the galactosyl donor is used. The relatively low concentration of galactosyl donor additionally reduces the degree of self-galactosylation of the donor, i.e. when the galactosyl group of a first galactosyl donor is transferred to a second galactosyl donor instead of to a galactosyl acceptor.

In some preferred embodiments of the invention, step c) comprises addition of further galactosyl donor to the mixture. This is particularly preferred when a relatively low concentration of the galactosyl donor is used. By adding more galactosyl donor one avoids the galactosyl donor being depleted in the mixture and the concentration of galactosyl donor may be controlled during the enzymatic reaction.

The addition of further galactosyl donor may involve discrete addition(s) of galactosyl donor, e.g. at least once during the enzymatic reaction. Alternatively, or additionally, the addition of further galactosyl donor may be a continuous addition during the enzymatic reaction. The further galactosyl donor is preferably of the same type as used in step a).

In some preferred embodiments of the invention, the concentration of galactosyl donor of the mixture during step c) is maintained at a concentration in the range of 0.01-1 mol/L, preferably in the range of 0.01-0.5 mol/L, and preferably in the range of 0.03-0.3 mol/L.

For example, the concentration of galactosyl donor of the mixture during step c) may be maintained at a concentration in the range of 0.02-0.1 mol/L.

Step c) may furthermore comprise addition of further galactosyl acceptor. This makes it possible to control the concentration of galactosyl acceptor of the mixture during step c) and e.g. to keep the galactosyl acceptor concentration substantially constant if this is desired.

In order to produce significant amounts of galacto-oligosaccharides, which contain two or three transferred galactosyl groups, the process should consume more galactosyl donor than galactosyl acceptor. Thereby more of the galactosyl acceptors will become galactosylated two or three times. Thus, in some preferred embodiments of the invention the molar ratio between the consumed galactosyl donor and the consumed galactosyl acceptor is at least 1:1, and preferably at least 5:1, and even more preferably at least 10:1.

Often it is required to enrich and/or purify the galacto-oligosaccharides of the composition and reduce the concentration of the galactosyl acceptor, the galactosyl donor and the released leaving group.

Thus, in some preferred embodiments of the invention the method furthermore comprises the step:

d) enriching the galacto-oligosaccharides of the composition of step c).

In the context of the present invention, the term "enriching the galacto-oligosaccharides" relates to increasing the relative amount of the galacto-oligosaccharides of the composition on a dry weight basis. This is typically done by removing some of the other solids of the composition, e.g. the lower saccharides, and optionally also the enzyme, if required.

The enrichment of step d) may for example involve chromatographic separation and/or nanofiltration. Details regarding such processes are described in Walstra *et al.* (2006) which is incorporated herein by reference for all purposes.

In some embodiments of the invention the enrichment involves that at least 50% (w/w on dry weight basis) of the molecules having a molar weight of at most 200 g/mol are removed from the composition of step c). For example, the enrichment may involve that at least 80% (w/w on dry weight basis) of the molecules having a molar weight of at most 200 g/mol are removed from the composition of step c).

In other embodiments of the invention the enrichment involves that at least 50% (w/w on dry weight basis) of the molecules having a molar weight of at most 350 g/mol are removed from the composition of step c). For example, the enrichment may involve that at least 80% (w/w on dry weight basis) of the molecules having a molar weight of at most 350 g/mol are removed from the composition of step c).

As an alternative, or in addition, to the enrichment it may be preferred that step d) comprises one or more processes which increase the concentration of the galacto-oligosaccharides in the composition. Examples of useful concentration steps are e.g. reverse osmosis, evaporation, and/or spray-drying.

The galacto-oligosaccharide-containing composition provided by the method may for example be in the form of a dry powder or in the form of a syrup.

The production of a dry powder typically requires one or more process steps, such as concentrating, evaporating, and/or spray-drying. Thus, in some preferred embodiments of the invention the step d) furthermore involves concentrating, evaporating, and/or spray-drying the composition in liquid form to obtain the composition in powder form. It is particularly preferred to spray-dry the liquid composition of step d) to obtain a powdered composition. Step d) may for example comprise the enrichment step followed by concentration step, e.g. nanofiltration, reverse osmosis, or evaporation, followed by a spray-drying step. Alternatively, step d) may comprise the concentration step followed by an enrichment step, followed by a spray-drying step. Concentrating the galacto-oligosaccharides of the composition prior to the enrichment may make the subsequent enrichment process more cost-efficient.

Efficient spray-drying may require addition of one or more auxiliary agent(s), such as maltodextrin, milk protein, caseinate, whey protein concentrate, and/or skimmed-milk powder.

The present process may e.g. be implemented as a batch process. The present process may alternatively be implemented as a fed-batch process. The present process may alternatively be implemented as a continuous process.

The present process may furthermore involve recirculation of enzyme and/or unused galactosyl acceptor back to the mixture. The recirculation may e.g. form part of step d). For example, step d) may involve separating galactosyl acceptor and/or the enzyme from the galacto-oligosaccharide-containing composition and recirculating galactosyl acceptor and/or enzyme to step a), or c). In the case of a batch process or a fed-batch process, the galactosyl acceptor and/or the enzyme may be recirculated to the mixture of the next batch.

In the case of a continuous process, the galactosyl acceptor may be recirculated back to part of the process line corresponding to step a) or step c). The enzyme may be recirculated back to part of the process line corresponding to step b) or step c).

It should be noted that the details and features related to steps a) and b) need not relate to the actual start of a production process, but should at least occur sometime during the process. However, in some embodiments of the invention the concentration of the galactosyl donor is kept within the range described in step a) during the entire duration of step c).

If the method is implemented as a batch or feed batch process, step a) preferably pertains to the composition of the mixture when the synthesis starts. If the method is a continuous process, step a) preferably pertains to the composition of the mixture during the synthesis under steady-state operation.

It may be perceived as desirable that the level of galactosylated galactosyl donor is kept as low as possible, as galactosylated galactosyl donor may be perceived as an undesired impurity, which is tricky to separate from the galactosylated galactosyl acceptor. In some preferred embodiments of the invention, the mixture of step a) contains at most 0.5 mol/L galactosylated galactosyl donor. The galactosylated galactosyl donor may for example contain at most 0.1 mol/L galactosylated galactosyl donor. Even more preferably the galactosylated galactosyl donor contains at most 0.01 mol/L galactosylated galactosyl donor, and preferably substantially no galactosylated galactosyl donor.

Described herein is also a composition comprising galacto-oligosaccharides, which composition is obtainable by the method as defined herein.

A further aspect decribed herin is a galacto-oligosaccharide-containing composition, e.g. the above-mentioned composition, said galacto-oligosaccharide-containing composition being obtainable according to the method described herein and comprising:

- a first galacto-oligosaccharide having the general formula Gal-X,
- a second galacto-oligosaccharide having the stoichiometric formula $(Gal)_2X$, such as e.g. the general formula Gal-Gal-X,
- a third galacto-oligosaccharide having the stoichiometric formula $(Gal)_3X$, such as e.g. the general formula Gal-Gal-Gal-X, and

wherein X is a glycosyl group, which is not lactosyl or glucosyl.

The galacto-oligosaccharide-containing composition described herein may for example be a food ingredient.

As described above, "X" or "-X" is preferably a glycosyl group of one of the galactosyl acceptors mentioned herein.

In some embodiments "X" or "-X" is a glycosyl group of a monosaccharide, which is not glucose. In other embodiments "-X" is a glycosyl group of a disaccharide, which is not lactose.

In some preferred embodiments of the invention "X" or "-X" is a fucosyl group. In other preferred embodiments of the invention "X" or "-X" is a galactosyl group.

In some preferred embodiments the galacto-oligosaccharide-containing composition has a molar ratio between:

- the total amount of the galacto-oligosaccharides Gal-X, $(Gal)_2X$, and $(Gal)_3X$, and
- the total amount of the galacto-oligosaccharides Gal-Glc, $Gal_2Glc$, Gal3Glc

of at least 5:95. For example, the above-mentioned molar ratio may be at least 1:4, preferably at least 1:1, and even more preferably at least 2:1. It may even be preferred that the above-mentioned molar ratio is at least 5:1, preferably at least 10:1, and even more preferably at least 20:1.

In other preferred embodiments the galacto-oligosaccharide-containing composition has a molar ratio between:

- the total amount of the galacto-oligosaccharides Gal-X, Gal-Gal-X, and Gal-Gal-Gal-X, and
- the total amount of the galacto-oligosaccharides Gal-Glc, Gal-Gal-Glc, Gal-Gal-Gal-Glc

of at least 5:95. For example, the above-mentioned molar ratio may be at least 1:4, preferably at least 1:1, and even more preferably at least 2:1. It may even be preferred that the above-mentioned molar ratio is at least 5:1, preferably at least 10:1, and even more preferably at least 20:1.

It is even possible that the galacto-oligosaccharide-containing composition does not contain any galacto-oligosaccharides of the formula Gal-Glc, Gal-Gal-Glc, and Gal-Gal-Gal-Glc at all.

In some embodiments the galacto-oligosaccharide-containing composition has a molar ratio between the first galacto-oligosaccharide, the second galacto-oligosaccharide, and the third galacto-oligosaccharide in the range of 50-99 : 1-45 : 0.5-25.

In other embodiments the galacto-oligosaccharide-containing composition has a molar ratio between the first galacto-oligosaccharide, the second galacto-oligosaccharide, and the third galacto-oligosaccharide in the range of 20-45 : 20-45: 20-45.

In further embodiments the galacto-oligosaccharide-containing composition has a molar ratio between the first galacto-oligosaccharide, the second galacto-oligosaccharide, and the third galacto-oligosaccharide in the range of 0.5-25 : 1-45 : 50-98.

In some preferred embodiments the galacto-oligosaccharide-containing composition comprises a total amount of the first galacto-oligosaccharide, second galacto-oligosaccharide, and third galacto-oligosaccharide of at least 10% by weight relative to the total weight of the galacto-oligosaccharide-containing composition. For example, the galacto-oligosaccharide-containing composition may comprise a total amount of the first galacto-oligosaccharide, second galacto-oligosaccharide, and third galacto-oligosaccharide of at least 20% by weight relative to the total weight of the galacto-oligosaccharide-containing composition, preferably at least 30% by weight, even more preferably at least 40% relative to the total weight of the galacto-oligosaccharide-containing composition.

Even higher levels of the first, second, and third galacto-oligosaccharides may be preferred. Thus, in some preferred embodiments the galacto-oligosaccharide-containing composition comprises a total amount of the first galacto-oligosaccharide, second galacto-oligosaccharide, and third galacto-oligosaccharide of at least 50% by weight relative to the total weight of the galacto-oligosaccharide-containing composition. For example, the galacto-oligosaccharide-containing composition may comprise a total amount of the first galacto-oligosaccharide, second galacto-oligosaccharide, and third galacto-oligosaccharide of at least 60% by weight relative to the total weight of the galacto-oligosaccharide-containing composition, preferably least 70% by weight, even more preferably at least 80% relative to the total weight of the galacto-oligosaccharide-containing composition.

Yet another aspect relates to a food product comprising the galacto-oligosaccharide-containing composition described herein.

In some embodiments the food product is a functional food product such as infant formula or a product for clinical nutrition.

In other embodiments the food product is a baked product, e.g. comprising baked dough, such as bread or similar products.

In further embodiments the food product is a dairy product, e.g. a fresh dairy product such as milk, or a fermented dairy product such as yoghurt.

In still further embodiments the food product is a pet food product.

It should be noted that embodiments and features described in the context of one of the aspects of the present invention also apply to the other aspects of the invention.

The invention will now be described in further details in the following non-limiting examples.

## EXAMPLES

Example 1: Preparation of the enzyme

A working volume of 750 mL fermentation medium was inoculated with a 2 mL starter-culture of Lysogeny broth (LB) medium with 100 mg/L ampicillin with an $OD_{600}$ of 3.0 grown for 12 hours. The fermentation was performed in EC medium containing 2 % (w/v) yeast extract, 2 % (w/v) soy peptone, 1 % (w/v) glucose and 100 mg/L ampicillin. The *E. coli* strain expressing OLGA347 β-galactosidase was prepared as described earlier (Jorgensen et al., US patent No. 6,555,348 B2, Examples 1 and 2). The fermentor was from Applikon with glass dished bottom vessels with a total volume of 2 L and equipped with two Rushton impellers. During the fermentation, pH was maintained at pH 6.5 by appropriate addition of 2 M NaOH and 2 M $H_3PO_4$ and temperature was controlled at 37 degrees C. Oxygen was supplied by bubbling with air at a rate of 1-2 L/min, and $pO_2$ was maintained at 30 % by increasing the agitation rate. Growth was followed by offline $OD_{600}$ readings. The culture was harvested by centrifugation after approximately 10 h of growth at an $OD_{600}$ value of 29.7. The 650 mL culture supernatant was stored at -20 degrees C. The periplasmic proteins were isolated from the cell pellet by osmotic shock by resuspending the cell pellet in 200 mL sucrose buffer (30 mM Tris-HCl, 40 % sucrose, 2 mM EDTA, pH 7.5) and incubating for 10 min at room temperature. After centrifugation, the supernatant was discarded and the pellet resuspended in 200 mL of cold water. 83 $\mu$L of a saturated $MgCl_2$ solution was added, and the supernatant containing the periplasmic proteins were collected by a centrifugation step. The periplasmic fraction was filter sterilized through a 0.2 $\mu$m Millipak 40 filter and stored at -20 degrees C.

The β-galactosidase activity of the 200 mL periplasmic fraction and the 650 mL culture supernatant was determined using *o*-nitrophenyl-*β*-D-galactopyranoside (OPNG) as a substrate according to protocol (J. Sambrook and D.W. Russell, Molecular Cloning - A laboratory manual, 3rd edition (2001), pp. 17.48-17.51). The majority of the activity was found in the periplasmic fraction (525 units, corresponding to 98%).

Example 2: Determination of the T-value of a beta-galactosidase enzyme

The T-value of a beta-galactosidase enzyme is determined according to the assay and formula given below.

Assay:

Prepare 3.3 mL enzyme solution consisting of the beta-galactosidase enzyme to be tested, 10 mM sodium citrate, 1 mM magnesium citrate, 1 mM calcium-citrate, Milli-Q water (Millipore, USA), and having a pH of 6.5. The enzyme solution should contain the beta-galactosidase enzyme in an amount sufficient to use 33% (w/w) of the added lactose in 1 hour under the present assay condition. The temperature of the enzyme solution should be 37 degrees C.

At time = $T_0$ 82.5 mg lactose monohydrate (for biochemistry, Merck Germany) is added to and mixed with, the enzyme solution, and the mixture is subsequently incubated at 37 degrees C for 4 hours. Precisely 1 hour after $T_0$ a 100 $\mu$L sample is collected and is diluted 1:5 with Milli-Q water and inactivated by heating to 85 °C for 10 min. The inactivated mixture is kept at -20 degrees C until the characterization.

Characterisation:

The determination of the amount (in mol) of produced galactose and the amount of used lactose (in mol) may be performed using any suitable analysis technique. For example, the diluted mixture may be analyzed by HPLC according to the method described by Richmond *et al.* (1982) and Simms *et al.* (1994). Other useful analysis techniques are described in El Razzi (2002).

Another example of a suitable analysis technique is ISO 5765-2:2002 (IDF 79-2: 2002) "Dried milk, dried ice-mixes and processed cheese - Determination of lactose content - Part 2: Enzymatic method utilizing the galactose moiety of the lactose".

Calculation of the T-value:

The T-value is calculated according to the following formula using the data obtained from the characterization of the diluted mixture of the assay:

$$\text{T-value} = \frac{\text{amount of produced galactose (in mol)}}{\text{amount of used lactose (in mol)}}$$

Example - T-value of the OLGA347 enzyme:

The above-mentioned assay was performed using the OLGA347 enzyme of Example 1.

The diluted mixture obtained from the assay was analyzed with respect to converted (i.e. used) lactose and generated galactose via analytical HPLC. The HPLC apparatus was from Waters and equipped with a differential refractometer (RI-detector) and a BioRad Aminex HPX-87C column (300x7.8 mm, 125-0055). Elution of saccharides was performed isocratically with 0.05 g/L CaAcetate, a flow rate of 0.3 mL/min. and an injection volume of 20 $\mu$L.

The obtained data was appropriately baseline corrected by automated software, peaks were individually identified and integrated. Quantification was performed by using external standards of lactose monohydrate (for biochemistry, Merck, Germany), D-(+)-glucose monohydrate (for biochemistry, Merck Eurolab, France), and D-(+)-galactose ($\geq$99%, Sigma-Aldrich, Italy).

The conversion of lactose and the formation of galactose to each time T was calculated from the quantified data. At time T=1h 29% of the lactose in the collected 100 $\mu$L sample had been converted, which corresponds to 2.3 $\mu$mol lactose. At time T=1 0.5 $\mu$mol galactose had been formed in the collected 100 $\mu$L sample. The T-value can therefore be calculated to 0.5 $\mu$mol / 2.3 $\mu$mol = 0.2.

The diluted mixture obtained from the assay was also analyzed with respect to converted (i.e. used) lactose and generated galactose via the enzymatic method ISO 5765-2. A Boehringer Mannheim Lactose/D-Galactose test-kit from R-Biopharm (Cat. No. 10 176 303 035) was used and the test performed according to protocol. The enzymatic method confirmed a T-value of the OLGA347-enzyme of 0.2.

Example - T-value of conventional lactase enzyme:

The above-mentioned assay was performed using the commercially available conventional lactase enzyme Lactozym Pure 2600L (Novozymes, Denmark). The diluted mixture obtained from the assay was analyzed as described for the OLGA347 enzyme. Tri- and tetra-saccharides were not present in detectable amounts and equal amounts of glucose and galactose were seen. The corresponding T-value is 1.

The T-values of commercially available beta-galactosidase from *Escherichia coli* (Product number: G6008, Sigma-Aldrich, Germany) and *Aspergillus oryzae* (Product number: G5160, Sigma-Aldrich, Germany) have also been determined, and both enzymes have a T-value of approx. 1.

Example 3: Synthesis of L-fucosyl-containing hetero-galacto-oligosaccharides by sequential addition of donor molecules

700 mg L-(-)-Fucose (99%, Sigma-Aldrich, Slovakia) and 20 mg lactose monohydrate (for biochemistry, Merck, Germany) was dissolved in 5 mL buffer (10 mM Na-Citrat, 20 mM $Na_2HPO_4$, pH 6.5) and maintained at a temperature of 37 degrees C. 2 mL OLGA347 enzyme, prepared as in Example 1, was added. This time is defined as T=0. During a 6 h period 20 mg lactose monohydrate was added with 30 min. intervals. 100 μL samples were acquired at times T=0, 1, 2, 3, 4, 5, and 7 h. Sample acquisition and characterization was done as in example 2. Mass spectrometry analysis was performed with an Agilent 1100 API-ES LC/MSD Quadropole scanning masses between 100 and 1000 amu (gas temperature: 350 °C, drying gas flow: 13.0 L/min, nebulizer pressure: 60 psig). The detected ions arise from complexation between the analyte and sodium cations from the solution, resulting in detected masses of $M(Na^+) = M + 23$ Da.

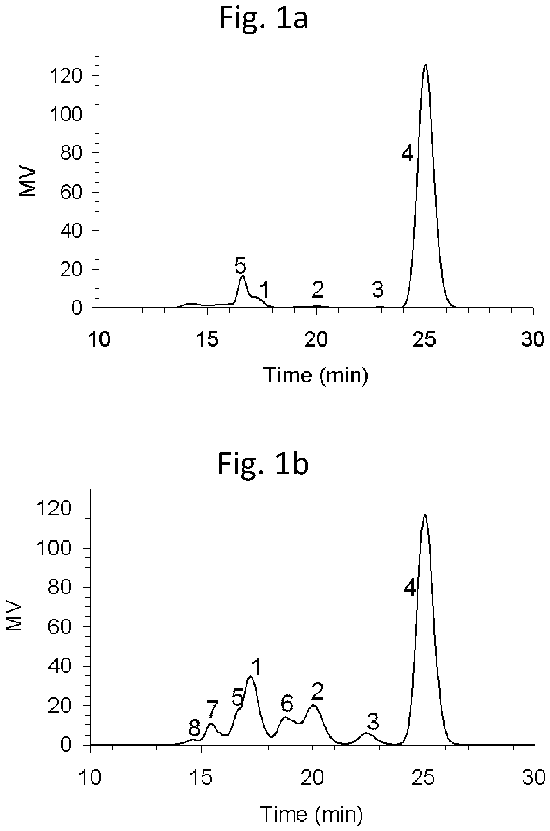

HPLC chromatograms from T=0 h and T=7 h are presented in Fig. 1a and Fig. 1b, respectively. The peaks have been identified by MS (data not shown) and are labeled in the figures. Legend: 1 = lactose, 2 = glucose, 3 = galactose, 4 = L-fucose, 5 = sucrose, 6 = Gal-Fuc disaccharides, 7 = Gal-Gal-Fuc & Gal-Gal-Glc trisaccharides, 8 = Gal-Gal-Gal-Fuc & Gal-Gal-Gal-Glc tetrasaccharides. In the figures it can be seen that the concentration of glucose and galactose increases with glucose being approximately 4 times more abundant than galactose. This is in accordance with the assumption that galactose is being used as donor molecule for the formation of galacto-oligosaccharides and the remaining glucose being released into solution. The concentration of lactose increases due to continuing addition of lactose. This peak also includes allolactose and Gal-Gal disaccharides formed in the enzymatic reaction. Furthermore, Gal-Fuc disaccharides, Gal-Gal-Glc and Gal-Gal-Fuc trisaccharides and Gal-Gal-Gal-Glc and Gal-Gal-Gal-Fuc tetrasaccharides are formed.

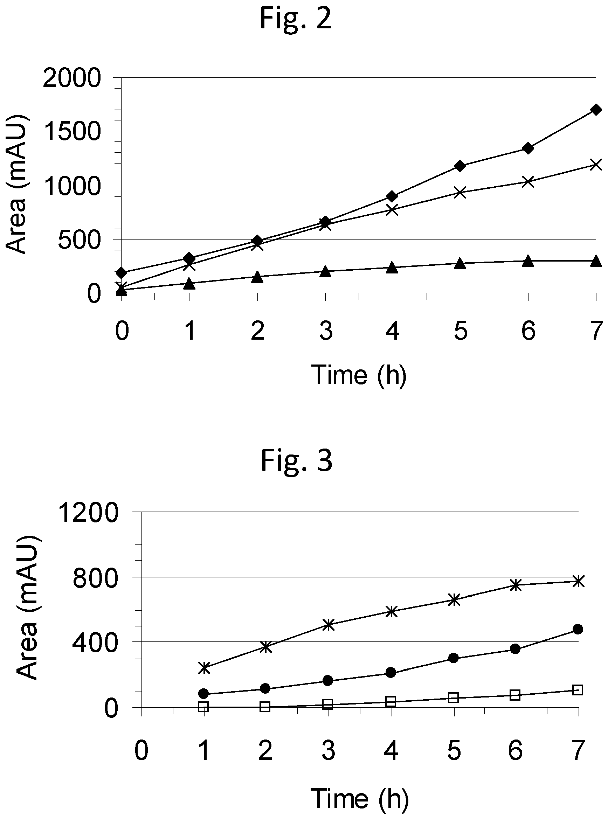

Plots of the calculated peak area as a function of time for lactose, glucose and galactose are presented in Fig. 2. Legend: diamond = lactose, cross = glucose, triangle = galactose. It is seen that the concentration of lactose, allolactose and Gal-Gal disaccharides increases and that the concentration of glucose increases linearly as glucose is being released into the solution during the enzymatic reaction. The concentration of galactose is low compared to glucose and the concentration increases only slowly. This indicates that almost all galactose provided from the cleavage of lactose has been used to form galacto-oligosaccharides. Furthermore, the concentration of L-fucose decreases during the course of the experiment, as L-fucose is being used as acceptor molecule in the enzymatic reaction to form galacto-oligosaccharides (data not shown).

Fig. 3 shows a plot of the calculated peak area as a function of time for the products from the enzymatic reaction. Legend: star = Gal-Fuc disaccharide, circle = Gal-Gal-Glc & Gal-Gal-Fuc trisaccharide, hollow square = Gal-Gal-Gal-Glc & Gal-Gal-Gal-Fuc tetrasaccharide. Both Gal-Fuc disaccharides and tri- and tetrasaccharides are formed. The trisaccharides are of the form Gal-Gal-Glc and Gal-Gal-Fuc, and the tetrasaccharides are of the form Gal-Gal-Gal-Glc and Gal-Gal-Gal-Fuc.

The concentration of Gal-Fuc disaccharides increases linearly and shows a tendency of reaching a plateau from T=6 h to T=7 h. The concentration of Gal-Gal-Glc and Gal-Gal-Fuc trisaccharides increases linearly and shows a tendency of exponential increase from T=4 h. The concentration of the Gal-Gal-Gal-Glc and Gal-Gal-Gal-Fuc tetrasaccharides increases linearly.

The amounts (w/w of total carbohydrate) of L-fucose-containing galacto-oligosaccharides are estimated based on HPLC and MS data at T=7 h: Gal-Fuc = 8 %, Gal-Gal-Fuc = 3 %, Gal-Gal-Gal-Fuc = 1 %. In all, L-fucose-containing galacto-oligosaccharides constitute 12 % after a reaction time of T=7 h. Upon removal of free L-fucose by chromatography, the calculated amount of L-fucose-containing galacto-oligosaccharides is 28 %.

Example 4: Synthesis of D-fucosyl-containing hetero-galacto-oligosaccharides

110 mg D-(+)-Fucose (≥98 %, Sigma-Aldrich, Slovakia) and 55 mg lactose monohydrate (for biochemistry, Merck, Germany) was dissolved in 1 mL buffer (10 mM Na-Citrat, 20 mM $Na_2HPO_4$, pH 6.5). 100 μL OLGA347 enzyme, which was prepared as in Example 1, was added. This time is defined as T=0. 100 μL samples were acquired at times T=0, 2, 4, 6, and 22 h. Sample acquisition and HPLC characterization was done as in example 2. MS characterization was done as in example 3.

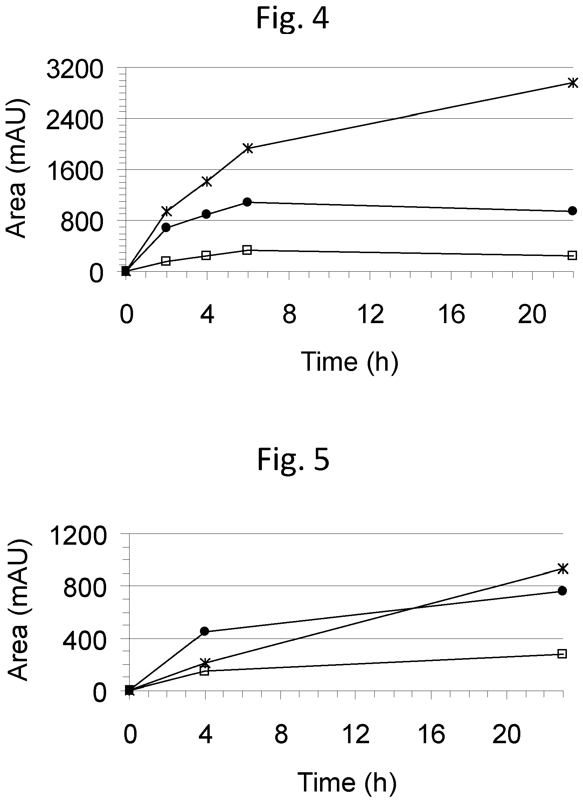

Fig. 4 shows a plot of the calculated peak area as a function of time for the products from the enzymatic reaction. Legend: star = Gal-Fuc disaccharide, circle = Gal-Gal-Glc & Gal-Gal-Fuc trisaccharide, hollow square = Gal-Gal-Gal-Glc & Gal-Gal-Gal-Fuc tetrasaccharide. Both Gal-Fuc disaccharides and tri- and tetrasaccharides are formed. The trisaccharides are of the form Gal-Gal-Glc and Gal-Gal-Fuc, and the tetrasaccharides are of the form Gal-Gal-Gal-Glc and Gal-Gal-Gal-Fuc.

The concentration of Gal-Fuc disaccharides shows the largest increase from T=0 to T=6 h. From T=6 to T=22 h the rate of increase is lower. The concentration of Gal-Gal-Glc and Gal-Gal-Fuc trisaccharides shows the second largest increase from T=0 to T=6 h. From T=6 to T=22 h the concentration drops to the level of T=4 h. The concentration of the Gal-Gal-Gal-Glc and Gal-Gal-Gal-Fuc tetrasaccharides increases from T=0 to T=6 h. From T=6 to T=22 h the concentration drops to the level of T=4 h.

The amounts (w/w of total carbohydrate) of D-fucose-containing galacto-oligosaccharides are estimated based on HPLC and MS data from T=22 h. Gal-Fuc = 20 %. Gal-Gal-Fuc = 6, and Gal-Gal-Gal-Fuc = 1 %. In all, D-fucose-containing galacto-oligosaccharides constitute 27 % after a reaction time of T=22 h. Upon removal of free D-fucose by chromatography, the calculated amount of D-fucose-containing galacto-oligosaccharides is 55 %.

Example 5: Synthesis of *N*-acetyl galactosamine-containing hetero-galacto-oligosaccharides

The experiment was conducted as in example 4, only with 110 mg *N*-Acetyl-D-galactosamine (GalNAc) (98%, Sigma-Aldrich, Germany) as acceptor molecule. 100 μL samples were acquired at times T=0, 4, and 23 h. Sample acquisition and HPLC characterization was done as in example 2. MS characterization was done as in example 3.

Fig. 5 shows a plot of the calculated peak area as a function of time for the products from the enzymatic reaction. Legend: star = Gal-GalNAc disaccharide, circle = Gal-Gal-Glc & Gal-Gal-GalNAc trisaccharide, hollow square = Gal-Gal-Gal-Glc & Gal-Gal-Gal-GalNAc tetrasaccharide. Both Gal-GalNAc disaccharides and tri- and tetrasaccharides are formed. The trisaccharides are of the form Gal-Gal-Glc and Gal-Gal-GalNAc, and the tetrasaccharides are of the form Gal-Gal-Gal-Glc and Gal-Gal-Gal-GalNAc.

The concentration of Gal-GalNAc disaccharides increases linearly from T=0 to T=23 h, and is the most abundant galacto-oligosaccharide at T=23 h. The concentration of Gal-Gal-Glc and Gal-Gal-GalNAc trisaccharides shows the largest increase from T=0 to T=4 h. From T=4 to T=23 h the rate of increase in concentration is lower and almost reaches the Gal-GalNAc level at T=23 h. The concentration of the Gal-Gal-Gal-Glc and Gal-Gal-Gal-Fuc tetrasaccharides increases from T=0 to T=23 h with the largest increase from T=0 to T=4 h. The amounts (w/w of total carbohydrate) of *N*-acetyl-galactosamine-containing galacto-oligosaccharides are estimated based on HPLC and MS data from T=23 h. Gal-GalNAc = 8 %, Gal-Gal-GalNAc = 5 %, and Gal-Gal-Gal-GalNAc = 2 %. In all, GalNAC-containing galacto-oligosaccharides constitute 15 % after a reaction time of T=23 h. Upon removal of free GalNAc by chromatography, the calculated amount of *N*-acetyl-galactosamine-containing galacto-oligosaccharides is 40 %.

Example 6: Synthesis of xylosyl-containing hetero-galacto-oligosaccharides

The experiment was conducted as in example 4, only with 110 mg D-(+)-Xylose (≥99 %, Sigma-Aldrich, USA) as acceptor molecule. 100 μL samples were acquired at times T=0, 5, and 23 h. Sample acquisition and HPLC characterization was done as in example 2. MS characterization was done as in example 3.

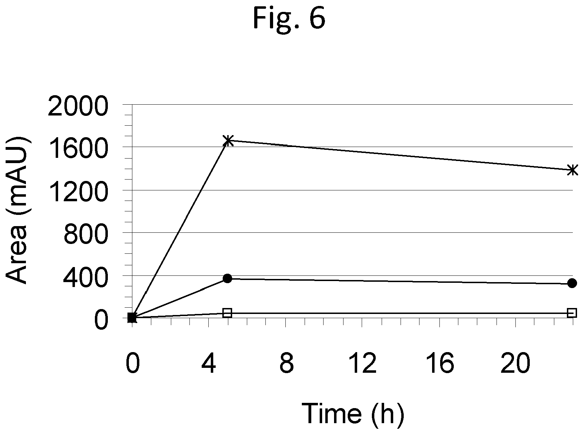

Fig. 6 shows a plot of the calculated peak area as a function of time for the products from the enzymatic reaction. Legend: star = Gal-Xyl disaccharide, circle = Gal-Gal-Glc & Gal-Gal-Xyl trisaccharide, hollow square = Gal-Gal-Gal-Glc & Gal-Gal-Gal-Xyl tetrasaccharide. Both Gal-Xyl disaccharides and tri- and tetrasaccharides are formed. The trisaccharides are of the form Gal-Gal-Glc and Gal-Gal-Xyl, and the tetrasaccharides are of the form Gal-Gal-Gal-Glc and Gal-Gal-Gal-Xyl.

The concentration of Gal-Xyl disaccharides shows the largest increase from T=0 to T=5 h. From T=5 to T=23 h the concentration drops to the level of T=4 h. The concentration of Gal-Gal-Glc and Gal-Gal-Xyl trisaccharides shows the second largest increase from T=0 to T=5 h. From T=5 to T=23 h the concentration drops to the level of T=4 h. The concentration of the Gal-Gal-Gal-Glc and Gal-Gal-Gal-Fuc tetrasaccharides increases from T=0 to T=5 h. From T=5 to T=23 h the concentration does not change at an observable level.

The amounts (w/w of total carbohydrate) of xylosyl-containing galacto-oligosaccharides are estimated based on HPLC and MS data from T=23 h. Gal-Xyl = 15 %. Gal-Gal-Xyl = 3 %. Gal-Gal-Gal-Xyl = 0.5 %. In all, xylosyl-containing galacto-oligosaccharides constitute 18.5 % after a reaction time of T=23 h. Upon removal of free Xylose by chromatography, the calculated amount of xylosyl-containing galacto-oligosaccharides is 41 %.

**REFERENCES**

| | |
|---|---|
| Buchholz (2005) | "Biocatalysts and Enzyme technology", Klaus Buchholz et al., ISBN-10: 3-527-30497-5, 2005, Wiley VCH Verlag GmbH |
| Franck (2002) | "Technological functionality of inulin and oligofructose", A. Franck, British Journal of Nutrition (2002), 87, Suppl. 2, S287-S291 |
| Yun (1996) | "Fructooligosaccharides-Occurrence, preparation, and application", J. W. Yun, Enzyme and Microbial Technology 19: 107-117, 1996 |
| Kunz (2000) | "Oligosaccharides in human milk: Structural, functional and metabolic aspects", Kunz et al., Ann. Rev. Nutr. 2000. 20:699-722 |

(continued)

| Simms *et al.* (1994) | Simms, P.J.; Hicks, K.B.; Haines, R.M.; Hotchkiss, A.T. and Osman, S.F.; (1994) Separations of lactose, lactobionic and lactobionolactose by high performance liquid chromatography. J. of Chromatography, 667, 67-73. |
| Richmond *et al.* (1982) | Richmond, M.L.; Barfuss, D.L.; Harte, B.R.; Gray, J.I. and Stine, C.M.; (1982) Separation of Carbohydrates in Dairy Products by High Performance Liquid Chromatography, J. of Dairy Science, 65 (8), 1394-1400. |
| El Razzi (2002) | "Carbohydrate Analysis by Modern Chromatography and Electrophoresis", volume 66, Journal of Chromatography Library, Elsevier Science, 2002, ISBN-10: 0444500618 |
| Walstra *et al.* (2006) | "Dairy science and technology", Walstra et al., CRC Press, Second edition, 2006 |

**Claims**

1. A method of producing a composition comprising one or more galacto-oligosaccharide(s), the method comprising the steps of:

    a) providing a mixture comprising

        - a galactosyl donor comprising a galactosyl group bound to a leaving group, which galactosyl donor has a molar weight of at most 350 g/mol,
        - a galactosyl acceptor which is different from the galactosyl donor, said galactosyl acceptor is a saccharide or a sugar-alcohol, and

    wherein the molar ratio between the galactosyl acceptor and the galactosyl donor is at least 1:10, and wherein the mixture comprises at least 0.05 mol/L of the galactosyl acceptor and the galactosyl donor in a concentration of at most 0.7 mol/L,
    b) providing an enzyme having beta-galactosidase activity and having a T-value of at most 0.6, said enzyme contacting the mixture, the determination of the T-value of a beta-galactosidase enzyme is performed by providing a solution consisting of the beta-galactosidase enzyme in an amount sufficient to use 33% (w/w) of added lactose in 1 hour at 37°C for 4 hours and calculated using the formula:

$$\text{T-value} = \frac{\text{amount of produced galactose (in mol)}}{\text{amount of used lactose (in mol)}}$$

    and
    c) allowing the enzyme to release the leaving group of the galactosyl donor and transfer the galactosyl group of the galactosyl donor to the galactosyl acceptor, thus forming the galacto-oligosaccharide, and thereby obtaining the composition comprising the galacto-oligosaccharide.

2. The method according to claim 1, wherein the enzyme has a T-value of at most 0.5.

3. The method according to claim 1 or 2, wherein the leaving group of the galactosyl donor is a glycosyl group.

4. The method according to any of the preceding claims, wherein the molar ratio between the galactosyl acceptor and the galactosyl donor is at least 1:5.

5. The method according to any of the preceding claims, wherein the mixture of step a) comprises the galactosyl donor in a concentration of at most 0.4 mol/L,

6. The method according to any of the preceding claims, wherein the galactosyl donor is lactose or lactitol.

7. The method according to any of the preceding claims, wherein step c) comprises addition of further galactosyl donor.

8. The method according to any of the preceding claims, wherein the concentration of galactosyl donor of the mixture during step c) is maintained at a concentration in the range of 0.01-1 mol/L.

9. The method according to any of the preceding claims furthermore comprising the step:
    d) enriching the galacto-oligosaccharide of the composition of step c).

10. The method according to claim 9, wherein the enrichment involves chromatographic separation and/or nanofiltration.


**Patentansprüche**

1. Verfahren zur Herstellung einer Zusammensetzung, die ein oder mehrere Galactooligosaccharide umfasst, wobei das Verfahren die Schritte umfasst:

    a) Bereitstellen eines Gemischs umfassend

- einen Galactosyldonator, der eine Galactosylgruppe umfasst, die an eine Abgangsgruppe gebunden ist, wobei der Galactosyldonator ein Molekulargewicht von höchstens 350 g/mol aufweist,
- einen Galaktosylakzeptor, der von dem Galactosyldonator verschieden ist, wobei der Galactosylakzeptor ein Saccharid oder ein Zuckeralkohol ist, und

wobei das Molverhältnis zwischen dem Galactosylakzeptor und dem Galactosyldonator wenigstens 1:10 beträgt und wobei das Gemisch wenigstens 0,05 mol/l an dem Galactosylakzeptor und den Galactosyldonator in einer Konzentration von höchstens 0,7 mol/l umfasst,

b) Bereitstellen eines Enzyms mit beta-Galactosidase-Aktivität und einem T-Wert von höchstens 0,6, wobei das Enzym mit dem Gemisch in Kontakt kommt, wobei die Bestimmung des T-Werts eines beta-Galactosidaseenzyms durch Bereitstellen einer Lösung, die aus dem beta-Galactosidaseenzym besteht, in einer Menge, die ausreicht, um 33 % (Gew./Gew.) von zugegebener Lactose in 1 Stunde bei 37 °C zu verbrauchen, für 4 Stunden durchgeführt und unter Verwendung der Formel

$$T\text{--Wert} = \frac{\text{Menge an erzeugter Galactose (in mol)}}{\text{Menge an verbrauchter Lactose (in mol)}}$$

berechnet wird,
und

c) Erlauben, dass das Enzym die Abgangsgruppe des Galactosyldonators freisetzt und die Galactosylgruppe des Galactosyldonators zu dem Galactosylakzeptor überführt und so das Galactooligosaccarid bildet, um dadurch die Zusammensetzung, die das Galactooligosaccharid umfasst, zu erhalten.

2. Verfahren gemäß Anspruch 1, wobei das Enzym einen T-Wert von höchstens 0,5 aufweist.

3. Verfahren gemäß Anspruch 1 oder 2, wobei die Abgangsgruppe des Galactosyldonators eine Glycosylgruppe ist.

4. Verfahren gemäß einem der vorstehenden Ansprüche, wobei das Molverhältnis zwischen dem Galactosylakzeptor und dem Galactosyldonator wenigstens 1:5 beträgt.

5. Verfahren gemäß einem der vorstehenden Ansprüche, wobei das Gemisch von Schritt a) den Galactosyldonator in einer Konzentration von höchstens 0,4 mol/l umfasst.

6. Verfahren gemäß einem der vorstehenden Ansprüche, wobei der Galactosyldonator Lactose oder Lactitol ist.

7. Verfahren gemäß einem der vorstehenden Ansprüche, wobei Schritt c) Zugeben von weiterem Galactosyldonator umfasst.

8. Verfahren gemäß einem der vorstehenden Ansprüche, wobei die Konzentration an Galactosyldonator in dem Gemisch bei Schritt c) bei einer Konzentration in dem Bereich von 0,01-1 mol/l gehalten wird.

9. Verfahren gemäß einem der vorstehenden Ansprüche, ferner umfassend den Schritt:
d) Anreichern des Galactooligosaccharids der Zusammensetzung von Schritt c).

10. Verfahren gemäß Anspruch 9, wobei das Anreichern chromatographische Trennung und/oder Nanofiltration umfasst.

**Revendications**

1. Procédé de production d'une composition comprenant un ou plusieurs galacto-oligosaccharides, le procédé comprenant les étapes consistant à :

a) fournir un mélange comprenant

- un donneur de galactosyle comprenant un groupe galactosyle lié à un groupe partant, lequel donneur de galactosyle a une masse molaire d'au maximum 350 g/mol,
- un accepteur de galactosyle qui est différent du donneur de galactosyle, ledit accepteur de galactosyle étant un saccharide ou un alcool de sucre, et

le rapport molaire entre l'accepteur de galactosyle et le donneur de galactosyle étant d'au moins 1:10 et le mélange comprenant au moins 0,05 mol/l de l'accepteur de galactosyle et le donneur de galactosyle en une concentration d'au maximum 0,7 mol/l,

b) fournir une enzyme ayant une activité de bêta-galactosidase et ayant une valeur de T d'au maximum 0,6, mettre ladite enzyme en contact avec le mélange, la détermination de la valeur de T d'une enzyme bêta-galactosidase est effectuée par le fourniture d'une solution constituée de l'enzyme bêta-galactosidase en une quantité suffisante pour utiliser 33 % (p/p) de lactose ajouté en 1 heure à 37 °C pendant 4 heures et calculée à l'aide de la formule :

$$\text{Valeur de T} = \frac{\text{quantité de galactose produit (en mol)}}{\text{quantité de lactose utilisé (en mol)}}$$

et

c) laisser l'enzyme libérer le groupe partant du donneur de galactosyle et transférer le groupe galactosyle du donneur de galactosyle vers l'accepteur de galactosyle, ce qui forme ainsi le galacto-oligosaccharide et permet de cette manière d'obtenir la composition comprenant le galacto-oligosaccharide.

2. Procédé selon la revendication 1, dans lequel l'enzyme a une valeur de T d'au maximum 0,5.

3. Procédé selon la revendication 1 ou 2, dans lequel le groupe partant du donneur de galactosyle est un groupe glycosyle.

4. Procédé selon l'une quelconque des revendications précédentes, dans lequel le rapport molaire entre l'accepteur de galactosyle et le donneur de galactosyle est d'au moins 1:5.

5. Procédé selon l'une quelconque des revendications précédentes, dans lequel le mélange de l'étape a) comprend le donneur de galactosyle en une concentration d'au maximum 0,4 mol/l.

6. Procédé selon l'une quelconque des revendications précédentes, dans lequel le donneur de galactosyle est le lactose ou le lactitol.

7. Procédé selon l'une quelconque des revendications précédentes, dans lequel l'étape c) comprend l'ajout de donneur de galactosyle supplémentaire.

8. Procédé selon l'une quelconque des revendications précédentes, dans lequel la concentration du donneur de galactosyle du mélange pendant l'étape c) est maintenue à une concentration dans la plage de 0,01-1 mol/l.

9. Procédé selon l'une quelconque des revendications précédentes comprenant en outre l'étape consistant à :
d) enrichir le galacto-oligosaccharide de la composition de l'étape c).

10. Procédé selon la revendication 9, dans lequel l'enrichissement met en jeu une séparation chromatographique et/ou une nanofiltration.

## Fig. 1a

## Fig. 1b

## Fig. 2

## Fig. 3

## Fig. 4

## Fig. 5

## Fig. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0190317 A2
- WO 0190317 A
- US 6555348 B2, Jorgensen

**Non-patent literature cited in the description**

- **DUMORTIER et al.** *Biotechnol Appl. Biochem,* 1994, vol. 19, 341-354
- **J. SAMBROOK ; D.W. RUSSELL.** Molecular Cloning - A laboratory manual. 2001, 17.48-17.51
- **KLAUS BUCHHOLZ et al.** Biocatalysts and Enzyme technology. Wiley VCH Verlag GmbH, 2005
- **A. FRANCK.** Technological functionality of inulin and oligofructose. *British Journal of Nutrition,* 2002, vol. 87 (2), S287-S291
- **J. W. YUN.** Fructooligosaccharides-Occurrence, preparation, and application. *Enzyme and Microbial Technology,* 1996, vol. 19, 107-117
- **KUNZ et al.** Oligosaccharides in human milk: Structural, functional and metabolic aspects. *Ann. Rev. Nutr.,* 2000, vol. 20, 699-722
- **SIMMS, P.J. ; HICKS, K.B. ; HAINES, R.M. ; HOTCHKISS, A.T. ; OSMAN, S.F.** Separations of lactose, lactobionic and lactobionolactose by high performance liquid chromatography. *J. of Chromatography,* 1994, vol. 667, 67-73
- **RICHMOND, M.L. ; BARFUSS, D.L. ; HARTE, B.R. ; GRAY, J.I. ; STINE, C.M.** Separation of Carbohydrates in Dairy Products by High Performance Liquid Chromatography. *J. of Dairy Science,* 1982, vol. 65 (8), 1394-1400
- Carbohydrate Analysis by Modern Chromatography and Electrophoresis. Journal of Chromatography Library. Elsevier Science, 2002, vol. 66
- **WALSTRA et al.** Dairy science and technology. CRC Press, 2006

SEQUENCE LISTING

<110> Arla Foods amba

<120> Galacto-oligosaccharide-containing composition and a method of producing it

<130> P1018PC00

<160> 2

<170> PatentIn version 3.1

<210> 1
<211> 5509
<212> DNA
<213> Bifidobacterium bifidum

<220>
<221> CDS
<222> (212)..(5470)
<223>

<400> 1

```
atgcgttgcg ttgcgatttt tccggccctg tatgggggat acaggattgg cgatggcgac          60

acgccgtttt tgttaatggc atttacatga aatacaggta atgagatatc attctcatga         120

tcaccgtgtg gatatcgcat tggtgcgtat acactaacag caacagagcg gcgcggcagg         180

cgctcgtgga ttcaatgaag aaggaacgtt t atg gca gtt cgc aga ctt ggt           232
                                    Met Ala Val Arg Arg Leu Gly
                                     1               5

ggc cgc atc gtg gct ttc gcc gcc aca gtg gcc ttg tca ata ccg tta          280
Gly Arg Ile Val Ala Phe Ala Ala Thr Val Ala Leu Ser Ile Pro Leu
         10              15              20

ggg ttg tta aca aat tca gcg tgg gcg gtc gag gac gcc acc cga tcc          328
Gly Leu Leu Thr Asn Ser Ala Trp Ala Val Glu Asp Ala Thr Arg Ser
 25              30              35

gac tcc acc acg cag atg agc tcc acg ccg gag gtg gtc tac tcc agc          376
Asp Ser Thr Thr Gln Met Ser Ser Thr Pro Glu Val Val Tyr Ser Ser
40              45              50              55

gcc gtg gat tcc aag cag aat cgc acc tcg gat ttc gac gcc aac tgg          424
Ala Val Asp Ser Lys Gln Asn Arg Thr Ser Asp Phe Asp Ala Asn Trp
             60              65              70

aag ttc atg ctg tcc gat tcc gtg cag gcg cag gat ccg gcg ttc gac          472
Lys Phe Met Leu Ser Asp Ser Val Gln Ala Gln Asp Pro Ala Phe Asp
             75              80              85

gat tcg gcc tgg cag cag gtc gac ctg ccg cat gac tac agc atc acg          520
Asp Ser Ala Trp Gln Gln Val Asp Leu Pro His Asp Tyr Ser Ile Thr
         90              95              100

cag aag tat tcg cag agc aac gag gcc gaa agc gca tac ctt ccc ggc          568
Gln Lys Tyr Ser Gln Ser Asn Glu Ala Glu Ser Ala Tyr Leu Pro Gly
     105              110              115

ggc acc ggc tgg tac cgc aag tcc ttc acc atc gac cgg gac ctc gcc          616
```

```
Gly Thr Gly Trp Tyr Arg Lys Ser Phe Thr Ile Asp Arg Asp Leu Ala
120             125             130             135

ggc aag cgc atc gcc atc aac ttc gac ggc gtg tac atg aac gcc acc    664
Gly Lys Arg Ile Ala Ile Asn Phe Asp Gly Val Tyr Met Asn Ala Thr
            140             145             150

gtc tgg ttc aac ggc gtc aag ctc ggc acc cat ccg tac ggc tac tcg    712
Val Trp Phe Asn Gly Val Lys Leu Gly Thr His Pro Tyr Gly Tyr Ser
            155             160             165

ccg ttc tcc ttc gac ctg acc ggc aac gcc aag ttc ggt ggg gag aac    760
Pro Phe Ser Phe Asp Leu Thr Gly Asn Ala Lys Phe Gly Gly Glu Asn
            170             175             180

acc atc gtc gtc aag gtc gag aac agg ctg ccg tcc agc cgc tgg tac    808
Thr Ile Val Val Lys Val Glu Asn Arg Leu Pro Ser Ser Arg Trp Tyr
            185             190             195

tcc ggc tcc ggc atc tac cgc gac gtc acc ctc acc gtc acc gac ggc    856
Ser Gly Ser Gly Ile Tyr Arg Asp Val Thr Leu Thr Val Thr Asp Gly
200             205             210             215

gtg cac gtc ggc aat aac ggc gtg gcc atc aag acc ccg agc ctc gcc    904
Val His Val Gly Asn Asn Gly Val Ala Ile Lys Thr Pro Ser Leu Ala
            220             225             230

acc caa aac ggc ggc gac gtg acg atg aac ctc acc acc aag gtc gcc    952
Thr Gln Asn Gly Gly Asp Val Thr Met Asn Leu Thr Thr Lys Val Ala
            235             240             245

aac gac acc gag gcc gcg gcg aac atc acc ctc aag cag acc gtg ttc   1000
Asn Asp Thr Glu Ala Ala Ala Asn Ile Thr Leu Lys Gln Thr Val Phe
            250             255             260

ccc aag gga ggc aag acc gac gcc gcc atc ggc acc gtc acc acc gca   1048
Pro Lys Gly Gly Lys Thr Asp Ala Ala Ile Gly Thr Val Thr Thr Ala
            265             270             275

tcc aag tcc atc gcg gcc ggt gcc agc gcg gac gtg acc tcc acg atc   1096
Ser Lys Ser Ile Ala Ala Gly Ala Ser Ala Asp Val Thr Ser Thr Ile
280             285             290             295

acc gcc gct tcg ccc aag ctg tgg agc atc aag aac ccg aac ctg tac   1144
Thr Ala Ala Ser Pro Lys Leu Trp Ser Ile Lys Asn Pro Asn Leu Tyr
            300             305             310

acc gtg cgc acc gaa gtg ctc aac ggc ggc aag gtg ctc gac act tac   1192
Thr Val Arg Thr Glu Val Leu Asn Gly Gly Lys Val Leu Asp Thr Tyr
            315             320             325

gac acc gaa tat ggc ttc cgc tgg acc ggc ttc gat gcg acc agc ggt   1240
Asp Thr Glu Tyr Gly Phe Arg Trp Thr Gly Phe Asp Ala Thr Ser Gly
            330             335             340

ttc tcg ctc aac ggc gag aaa gtc aag ctc aag ggc gtc tca atg cat   1288
Phe Ser Leu Asn Gly Glu Lys Val Lys Leu Lys Gly Val Ser Met His
            345             350             355

cat gac cag gga tcg ctc ggc gcg gtc gcc aac cgc cgc gcc atc gag   1336
His Asp Gln Gly Ser Leu Gly Ala Val Ala Asn Arg Arg Ala Ile Glu
360             365             370             375
```

```
cgc cag gtc gag att ctc cag aag atg ggc gtc aac tcg atc cgc acc    1384
Arg Gln Val Glu Ile Leu Gln Lys Met Gly Val Asn Ser Ile Arg Thr
            380                 385                 390

acg cac aac ccc gca gcc aag gcg ctg att gac gtc tgc aac gag aag    1432
Thr His Asn Pro Ala Ala Lys Ala Leu Ile Asp Val Cys Asn Glu Lys
            395                 400                 405

ggc gtc ctc gtg gtc gaa gag gtc ttc gac atg tgg aac cgg tcg aag    1480
Gly Val Leu Val Val Glu Glu Val Phe Asp Met Trp Asn Arg Ser Lys
            410                 415                 420

aac ggc aac acc gag gat tac ggc aag tgg ttc ggc cag gcc atc gcc    1528
Asn Gly Asn Thr Glu Asp Tyr Gly Lys Trp Phe Gly Gln Ala Ile Ala
            425                 430                 435

ggt gac aac gcc gtc ctg ggt ggc gac aag gac gag acc tgg gcc aag    1576
Gly Asp Asn Ala Val Leu Gly Gly Asp Lys Asp Glu Thr Trp Ala Lys
440                 445                 450                 455

ttc gac ctg acc agc acc atc aac cgt gac agg aac gcc ccg tcc gtc    1624
Phe Asp Leu Thr Ser Thr Ile Asn Arg Asp Arg Asn Ala Pro Ser Val
            460                 465                 470

atc atg tgg tcg ctc ggc aac gag atg atg gaa ggc atc agc ggc agc    1672
Ile Met Trp Ser Leu Gly Asn Glu Met Met Glu Gly Ile Ser Gly Ser
            475                 480                 485

gtc tcg ggc ttc ccg gct acc tcc gcc aag ctg gtc gca tgg acg aag    1720
Val Ser Gly Phe Pro Ala Thr Ser Ala Lys Leu Val Ala Trp Thr Lys
            490                 495                 500

gcc gcg gac agc acc cgc ccg atg acc tac ggc gac aac aag atc aag    1768
Ala Ala Asp Ser Thr Arg Pro Met Thr Tyr Gly Asp Asn Lys Ile Lys
            505                 510                 515

gcc aac tgg aac gag tcg aac acc atg ggc gac aac ctg acc gcc aac    1816
Ala Asn Trp Asn Glu Ser Asn Thr Met Gly Asp Asn Leu Thr Ala Asn
520                 525                 530                 535

ggc ggc gtg gtc ggc acc aac tac tcc gac ggc gcg aac tac gac aag    1864
Gly Gly Val Val Gly Thr Asn Tyr Ser Asp Gly Ala Asn Tyr Asp Lys
            540                 545                 550

atc cgc acg acc cac ccc tca tgg gcc atc tat ggt tcc gag acg gcg    1912
Ile Arg Thr Thr His Pro Ser Trp Ala Ile Tyr Gly Ser Glu Thr Ala
            555                 560                 565

tcc gcc atc aac agc cga ggc atc tac aac cgc acc acc ggc ggc gcc    1960
Ser Ala Ile Asn Ser Arg Gly Ile Tyr Asn Arg Thr Thr Gly Gly Ala
            570                 575                 580

cag tca agc gac aag cag ctg acc agc tat gac aat tcc gca gtc ggc    2008
Gln Ser Ser Asp Lys Gln Leu Thr Ser Tyr Asp Asn Ser Ala Val Gly
            585                 590                 595

tgg ggc gcc gtc gcc agc tcc gcc tgg tac gac gtg gtc cag cgc gat    2056
Trp Gly Ala Val Ala Ser Ser Ala Trp Tyr Asp Val Val Gln Arg Asp
600                 605                 610                 615

ttc gtc gcc ggc aca tac gtg tgg acc ggc ttc gac tac ctc ggc gaa    2104
Phe Val Ala Gly Thr Tyr Val Trp Thr Gly Phe Asp Tyr Leu Gly Glu
            620                 625                 630
```

```
ccc acc ccg tgg aac ggc acc ggc tcc ggc gcc gtg ggc tcc ttg gcc      2152
Pro Thr Pro Trp Asn Gly Thr Gly Ser Gly Ala Val Gly Ser Leu Ala
            635                 640                 645

gtc gcc gaa gaa ctc gta ctt cgg cat cgt cga cac cgc agg ctt ccc      2200
Val Ala Glu Glu Leu Val Leu Arg His Arg Arg His Arg Arg Leu Pro
            650                 655                 660

gaa gac acc tat tac ttc tat cag agc cag tgg aac gac gac gtg cac      2248
Glu Asp Thr Tyr Tyr Phe Tyr Gln Ser Gln Trp Asn Asp Asp Val His
            665                 670                 675

acg ctg cac atc ctc ccc gca tgg aac gag aac gtc gtc gcc aag ggc      2296
Thr Leu His Ile Leu Pro Ala Trp Asn Glu Asn Val Val Ala Lys Gly
680                 685                 690                 695

tcc ggc aac aac gtg ccg gtc gtc gtc tac acc gac gcg gcc aag gtc      2344
Ser Gly Asn Asn Val Pro Val Val Val Tyr Thr Asp Ala Ala Lys Val
                700                 705                 710

aag ctg tac ttc aca ccg aag ggc agt acc gaa aag cga ctg atc gga      2392
Lys Leu Tyr Phe Thr Pro Lys Gly Ser Thr Glu Lys Arg Leu Ile Gly
            715                 720                 725

gag aag tcc ttc acc aag aag acc acc gcg gcc gga tac acc tat cag      2440
Glu Lys Ser Phe Thr Lys Lys Thr Thr Ala Ala Gly Tyr Thr Tyr Gln
            730                 735                 740

gtc tac gag ggc tcc gac aag gac tcc acc gcc cac aag aac atg tac      2488
Val Tyr Glu Gly Ser Asp Lys Asp Ser Thr Ala His Lys Asn Met Tyr
            745                 750                 755

ctg acc tgg aac gtg ccg tgg gcc gag ggc acc atc tcc gcc gaa gca      2536
Leu Thr Trp Asn Val Pro Trp Ala Glu Gly Thr Ile Ser Ala Glu Ala
760                 765                 770                 775

tac gac gag aac aac agg ctg atc ccc gag ggg tcc acc gag ggc aac      2584
Tyr Asp Glu Asn Asn Arg Leu Ile Pro Glu Gly Ser Thr Glu Gly Asn
                780                 785                 790

gcg tcg gtg acc acc acc ggc aag gcc gcg aag ctt aaa gcc gat gcc      2632
Ala Ser Val Thr Thr Thr Gly Lys Ala Ala Lys Leu Lys Ala Asp Ala
                795                 800                 805

gac cgc aag acg atc acc gcg gac ggc aag gac ctg tcg tac atc gag      2680
Asp Arg Lys Thr Ile Thr Ala Asp Gly Lys Asp Leu Ser Tyr Ile Glu
            810                 815                 820

gtc gac gtg acc gac gcc aac ggc cat atc gtc ccc gat gcc gcc aac      2728
Val Asp Val Thr Asp Ala Asn Gly His Ile Val Pro Asp Ala Ala Asn
            825                 830                 835

cgc gtc acc ttc gac gtc aag ggc gcc ggc aaa ctg gtc ggc gtc gac      2776
Arg Val Thr Phe Asp Val Lys Gly Ala Gly Lys Leu Val Gly Val Asp
840                 845                 850                 855

aac ggc agc tcg ccg gat cac gac tcc tat cag gcc gac aac cgc aag      2824
Asn Gly Ser Ser Pro Asp His Asp Ser Tyr Gln Ala Asp Asn Arg Lys
                860                 865                 870

gcg ttc agc ggc aag gtg ctc gcc atc gtc cag tcc acc aag gag gcg      2872
Ala Phe Ser Gly Lys Val Leu Ala Ile Val Gln Ser Thr Lys Glu Ala
```

875             880             885

```
ggc gag atc acc gtc acc gcc aag gcc gac ggt ctg caa tca tcc aca      2920
Gly Glu Ile Thr Val Thr Ala Lys Ala Asp Gly Leu Gln Ser Ser Thr
        890                 895                 900

gtg aag atc gcc acc acc gcc gtc ccc ggc acc agc acc gag aag acg      2968
Val Lys Ile Ala Thr Thr Ala Val Pro Gly Thr Ser Thr Glu Lys Thr
        905                 910                 915

gtc cgc agc ttc tac tac tcg cgc aac tac tac gtc aag acc ggc aac      3016
Val Arg Ser Phe Tyr Tyr Ser Arg Asn Tyr Tyr Val Lys Thr Gly Asn
920                 925                 930                 935

aag ccg att ctg ccg agt gat gtc gag gtg cgc tac tcc gac ggc acg      3064
Lys Pro Ile Leu Pro Ser Asp Val Glu Val Arg Tyr Ser Asp Gly Thr
            940                 945                 950

tcg gac cgt cag aac gtc aca tgg gac gca gtc agc gac gac cag atc      3112
Ser Asp Arg Gln Asn Val Thr Trp Asp Ala Val Ser Asp Asp Gln Ile
            955                 960                 965

gcc aag gcc ggt tcg ttc agc gtg gcc ggc acg gtc gcc ggg cag aag      3160
Ala Lys Ala Gly Ser Phe Ser Val Ala Gly Thr Val Ala Gly Gln Lys
            970                 975                 980

atc tcc gtg cgc gtg acg atg atc gac gag atc ggt gcg ctg ctc aac      3208
Ile Ser Val Arg Val Thr Met Ile Asp Glu Ile Gly Ala Leu Leu Asn
            985                 990                 995

tat tcg gcc agc aca ccg  gtc ggc acg ccc gcc  gtg ctg cct ggc        3253
Tyr Ser Ala Ser Thr Pro  Val Gly Thr Pro Ala  Val Leu Pro Gly
1000                1005                 1010

tcg cgt ccg gcc gtg ctg  ccc gac ggc acc gtg  acc agc gcg aac        3298
Ser Arg Pro Ala Val Leu  Pro Asp Gly Thr Val  Thr Ser Ala Asn
1015                1020                 1025

ttc gcc gtc cac tgg acc  aag ccc gcc gac acc  gtg tac aac acg        3343
Phe Ala Val His Trp Thr  Lys Pro Ala Asp Thr  Val Tyr Asn Thr
1030                1035                 1040

gcc ggc acc gtc aag gtc  ccc ggc acc gcc acc  gtc ttc ggc aag        3388
Ala Gly Thr Val Lys Val  Pro Gly Thr Ala Thr  Val Phe Gly Lys
1045                1050                 1055

gag ttc aag gtc acc gcg  acg att cgc gtg cag  cgg tcg cag gtc        3433
Glu Phe Lys Val Thr Ala  Thr Ile Arg Val Gln  Arg Ser Gln Val
1060                1065                 1070

acc atc ggc agc agc gtc  tcc ggc aat gcg ctg  cgc ctg act cag        3478
Thr Ile Gly Ser Ser Val  Ser Gly Asn Ala Leu  Arg Leu Thr Gln
1075                1080                 1085

aac atc ccc gcc gac aag  cag tcc gac acg ctg  gac gcc atc aag        3523
Asn Ile Pro Ala Asp Lys  Gln Ser Asp Thr Leu  Asp Ala Ile Lys
1090                1095                 1100

gac ggc tcc acg acc gtc  gac gcc aat acc ggc  ggc ggc gcg aac        3568
Asp Gly Ser Thr Thr Val  Asp Ala Asn Thr Gly  Gly Gly Ala Asn
1105                1110                 1115

ccg tca gca tgg acc aac  tgg gcg tac tcg aag  gcc ggc cac aac        3613
```

```
Pro  Ser Ala Trp Thr Asn  Trp Ala Tyr Ser Lys  Ala Gly His Asn
1120                 1125                1130

acc  gcc gag atc acc ttc  gag tac gcg acc gag  cag cag ctc ggc      3658
Thr  Ala Glu Ile Thr Phe  Glu Tyr Ala Thr Glu  Gln Gln Leu Gly
1135                 1140                1145

cag  att gtc atg tac ttc  ttc cgc gac agc aac  gcg gtg agg ttc      3703
Gln  Ile Val Met Tyr Phe  Phe Arg Asp Ser Asn  Ala Val Arg Phe
1150                 1155                1160

ccc  gac gcc ggc aag acg  aag atc cag atc tcc  gcg gac ggc aag      3748
Pro  Asp Ala Gly Lys Thr  Lys Ile Gln Ile Ser  Ala Asp Gly Lys
1165                 1170                1175

aac  tgg acg gat ctc gct  gcc acg gag acc atc  gcg gcc cag gag      3793
Asn  Trp Thr Asp Leu Ala  Ala Thr Glu Thr Ile  Ala Ala Gln Glu
1180                 1185                1190

tcg  tcc gac cga gtc aag  ccg tac acc tat gac  ttc gct ccg gtg      3838
Ser  Ser Asp Arg Val Lys  Pro Tyr Thr Tyr Asp  Phe Ala Pro Val
1195                 1200                1205

gga  gcc acg ttc gtc aag  gtc acg gtc acc aac  gcc gac acc aca      3883
Gly  Ala Thr Phe Val Lys  Val Thr Val Thr Asn  Ala Asp Thr Thr
1210                 1215                1220

acc  ccc agc ggc gtg gtc  tgc gcc ggc ctg acc  gag atc gag ctg      3928
Thr  Pro Ser Gly Val Val  Cys Ala Gly Leu Thr  Glu Ile Glu Leu
1225                 1230                1235

aag  acc gcg acc agc aag  ttc gtc acg aac acg  tcc gcc gcg ctc      3973
Lys  Thr Ala Thr Ser Lys  Phe Val Thr Asn Thr  Ser Ala Ala Leu
1240                 1245                1250

tcg  tcg ctg aca gtg aac  ggc acg aag gtc tcc  gac tcc gtg ctc      4018
Ser  Ser Leu Thr Val Asn  Gly Thr Lys Val Ser  Asp Ser Val Leu
1255                 1260                1265

gcc  gcc ggc tcc tac aac  acg ccc gcg atc atc  gcg gac gtc aaa      4063
Ala  Ala Gly Ser Tyr Asn  Thr Pro Ala Ile Ile  Ala Asp Val Lys
1270                 1275                1280

gcc  gag ggc gaa ggc aac  gcc agc gtc acc gtg  ctg ccc gcg cac      4108
Ala  Glu Gly Glu Gly Asn  Ala Ser Val Thr Val  Leu Pro Ala His
1285                 1290                1295

gac  aac gtg atc cgc gtg  atc acc gag tcc gag  gac cac gtc acg      4153
Asp  Asn Val Ile Arg Val  Ile Thr Glu Ser Glu  Asp His Val Thr
1300                 1305                1310

cgc  aag acc ttc acc atc  aac ctg ggc acg gag  cag gaa ttc ccc      4198
Arg  Lys Thr Phe Thr Ile  Asn Leu Gly Thr Glu  Gln Glu Phe Pro
1315                 1320                1325

gca  gac tcc gat gaa cgc  gac tac ccg gcc gcc  gac atg acg gtc      4243
Ala  Asp Ser Asp Glu Arg  Asp Tyr Pro Ala Ala  Asp Met Thr Val
1330                 1335                1340

acc  gtg ggc agc gaa cag  acg tcc ggc acc gcg  acc gaa ggc ccg      4288
Thr  Val Gly Ser Glu Gln  Thr Ser Gly Thr Ala  Thr Glu Gly Pro
1345                 1350                1355
```

```
aag  aaa  ttc  gcg  gtc  gac  ggc  aac  acc  agc  acg  tac  tgg  cat  tcc          4333
Lys  Lys  Phe  Ala  Val  Asp  Gly  Asn  Thr  Ser  Thr  Tyr  Trp  His  Ser
1360                     1365                1370

aac  tgg  acg  ccc  acc  acc  gtg  aac  gac  ctg  tgg  atc  gcc  ttc  gag          4378
Asn  Trp  Thr  Pro  Thr  Thr  Val  Asn  Asp  Leu  Trp  Ile  Ala  Phe  Glu
1375                     1380                1385

ctc  cag  aaa  ccc  acc  aag  ctc  gac  gcg  ctg  cgc  tac  ctg  ccg  cgc          4423
Leu  Gln  Lys  Pro  Thr  Lys  Leu  Asp  Ala  Leu  Arg  Tyr  Leu  Pro  Arg
1390                     1395                1400

ccc  gcg  ggc  agc  aag  aac  ggc  tcc  gtc  acc  gaa  tac  aag  gtt  cag          4468
Pro  Ala  Gly  Ser  Lys  Asn  Gly  Ser  Val  Thr  Glu  Tyr  Lys  Val  Gln
1405                     1410                1415

gtc  agc  gat  gac  ggc  acc  aac  tgg  acc  gac  gcg  ggc  tcc  ggc  aca          4513
Val  Ser  Asp  Asp  Gly  Thr  Asn  Trp  Thr  Asp  Ala  Gly  Ser  Gly  Thr
1420                     1425                1430

tgg  acc  acc  gat  tac  ggc  tgg  aag  ctc  gcc  gag  ttc  aat  cag  ccg          4558
Trp  Thr  Thr  Asp  Tyr  Gly  Trp  Lys  Leu  Ala  Glu  Phe  Asn  Gln  Pro
1435                     1440                1445

gtg  acc  acc  aag  cac  gtg  cgg  ctc  aag  gcc  gtc  cac  acc  tat  gcg          4603
Val  Thr  Thr  Lys  His  Val  Arg  Leu  Lys  Ala  Val  His  Thr  Tyr  Ala
1450                     1455                1460

gat  tcc  ggc  aac  gac  aag  ttc  atg  tcc  gcc  tcc  gaa  atc  cgc  ctg          4648
Asp  Ser  Gly  Asn  Asp  Lys  Phe  Met  Ser  Ala  Ser  Glu  Ile  Arg  Leu
1465                     1470                1475

cgc  aag  gcc  gtc  gac  acc  acc  gac  atc  agc  ggc  gcg  acc  gtg  acc          4693
Arg  Lys  Ala  Val  Asp  Thr  Thr  Asp  Ile  Ser  Gly  Ala  Thr  Val  Thr
1480                     1485                1490

gtg  ccc  gcc  aag  ctg  acc  gtc  gac  cgg  gtg  gac  gcc  gac  cat  ccc          4738
Val  Pro  Ala  Lys  Leu  Thr  Val  Asp  Arg  Val  Asp  Ala  Asp  His  Pro
1495                     1500                1505

gcc  acc  ttc  gcc  acg  aag  gac  gtg  acg  gtg  acg  ttg  ggc  gac  gcc          4783
Ala  Thr  Phe  Ala  Thr  Lys  Asp  Val  Thr  Val  Thr  Leu  Gly  Asp  Ala
1510                     1515                1520

acg  ctg  cgc  tac  ggc  gtg  gac  tac  ctg  ctc  gac  tac  gcg  ggc  aac          4828
Thr  Leu  Arg  Tyr  Gly  Val  Asp  Tyr  Leu  Leu  Asp  Tyr  Ala  Gly  Asn
1525                     1530                1535

acc  gcc  gtc  ggc  aag  gcc  acg  gtg  acc  gtg  cgc  ggc  atc  gac  aag          4873
Thr  Ala  Val  Gly  Lys  Ala  Thr  Val  Thr  Val  Arg  Gly  Ile  Asp  Lys
1540                     1545                1550

tac  tcc  ggc  acc  gtc  gcc  aag  acg  ttc  acc  atc  gaa  ctg  aag  aac          4918
Tyr  Ser  Gly  Thr  Val  Ala  Lys  Thr  Phe  Thr  Ile  Glu  Leu  Lys  Asn
1555                     1560                1565

gcc  ccg  gcg  ccg  gaa  ccg  acg  ctg  acc  tcg  gtg  agc  gtc  aag  acc          4963
Ala  Pro  Ala  Pro  Glu  Pro  Thr  Leu  Thr  Ser  Val  Ser  Val  Lys  Thr
1570                     1575                1580

aag  cct  tcc  aag  ctg  acc  tat  gtg  gtc  ggc  gac  gcg  ttc  gac  ccg          5008
Lys  Pro  Ser  Lys  Leu  Thr  Tyr  Val  Val  Gly  Asp  Ala  Phe  Asp  Pro
1585                     1590                1595
```

23

```
gca   gga  ctg  gtg  ctg  cag   cac  gac  aga  cag  gcc   gat  cgc  ccc  cca          5053
Ala   Gly  Leu  Val  Leu  Gln   His  Asp  Arg  Gln  Ala   Asp  Arg  Pro  Pro
1600                      1605                    1610

cag   cca  ctt  gtt  gga  gaa   cag  gcc  gac  gaa  cgc   gga  ctg  acg  tgc          5098
Gln   Pro  Leu  Val  Gly  Glu   Gln  Ala  Asp  Glu  Arg   Gly  Leu  Thr  Cys
1615                      1620                    1625

gga   acg  cga  tgc  gat  cgc   gtt  gaa  cag  ctg  cgc   aaa  cac  gag  aat          5143
Gly   Thr  Arg  Cys  Asp  Arg   Val  Glu  Gln  Leu  Arg   Lys  His  Glu  Asn
1630                      1635                    1640

cgt   gaa  gcc  cat  cgt  acg   ggc  ctc  gat  cat  ctg   gaa  ttc  gtg  ggt          5188
Arg   Glu  Ala  His  Arg  Thr   Gly  Leu  Asp  His  Leu   Glu  Phe  Val  Gly
1645                      1650                    1655

gcc   gcc  gat  gga  gcg  gtc   ggt  gaa  cag  gcc  acc   ttc  aag  gtg  cat          5233
Ala   Ala  Asp  Gly  Ala  Val   Gly  Glu  Gln  Ala  Thr   Phe  Lys  Val  His
1660                      1665                    1670

gtc   cat  gcc  gat  caa  ggt   gac  ggc  cgc  cat  gat   gat  gcc  gat  gaa          5278
Val   His  Ala  Asp  Gln  Gly   Asp  Gly  Arg  His  Asp   Asp  Ala  Asp  Glu
1675                      1680                    1685

cgc   gat  atc  gat  cca  cat   gtc  cct  gtc  gat  cac   gcg  gtc  ggt  gag          5323
Arg   Asp  Ile  Asp  Pro  His   Val  Pro  Val  Asp  His   Ala  Val  Gly  Glu
1690                      1695                    1700

ctt   gcg  cgg  gct  gcg  tgc   cat  cac  gtc  atc  ggt   ctg  cgg  gtc  gac          5368
Leu   Ala  Arg  Ala  Ala  Cys   His  His  Val  Ile  Gly   Leu  Arg  Val  Asp
1705                      1710                    1715

acc   cat  cgc  ctc  aag  gca   tcc  ggc  ttc  cag  atc   ccc  gcc  gac  gac          5413
Thr   His  Arg  Leu  Lys  Ala   Ser  Gly  Phe  Gln  Ile   Pro  Ala  Asp  Asp
1720                      1725                    1730

atg   gcc  gag  atc  gac  cgc   atc  acc  ggc  ttc  cac   cgc  ttc  gag  cgc          5458
Met   Ala  Glu  Ile  Asp  Arg   Ile  Thr  Gly  Phe  His   Arg  Phe  Glu  Arg
1735                      1740                    1745

cac   gtc  ggc  tga  cgtgattggg  cttccccgct  gtctggtgcc  ggctcgcga               5509
His   Val  Gly
1750
```

<210> 2
<211> 1752
<212> PRT
<213> Bifidobacterium bifidum


<400> 2

Met Ala Val Arg Arg Leu Gly Gly Arg Ile Val Ala Phe Ala Ala Thr
1                   5                   10                  15

Val Ala Leu Ser Ile Pro Leu Gly Leu Leu Thr Asn Ser Ala Trp Ala
                20                  25                  30

Val Glu Asp Ala Thr Arg Ser Asp Ser Thr Thr Gln Met Ser Ser Thr
                35                  40                  45

```
Pro Glu Val Val Tyr Ser Ser Ala Val Asp Ser Lys Gln Asn Arg Thr
    50              55              60

Ser Asp Phe Asp Ala Asn Trp Lys Phe Met Leu Ser Asp Ser Val Gln
65              70              75                      80

Ala Gln Asp Pro Ala Phe Asp Asp Ser Ala Trp Gln Gln Val Asp Leu
                85              90                      95

Pro His Asp Tyr Ser Ile Thr Gln Lys Tyr Ser Gln Ser Asn Glu Ala
            100             105             110

Glu Ser Ala Tyr Leu Pro Gly Gly Thr Gly Trp Tyr Arg Lys Ser Phe
    115             120             125

Thr Ile Asp Arg Asp Leu Ala Gly Lys Arg Ile Ala Ile Asn Phe Asp
    130             135             140

Gly Val Tyr Met Asn Ala Thr Val Trp Phe Asn Gly Val Lys Leu Gly
145             150             155             160

Thr His Pro Tyr Gly Tyr Ser Pro Phe Ser Phe Asp Leu Thr Gly Asn
            165             170             175

Ala Lys Phe Gly Gly Glu Asn Thr Ile Val Val Lys Val Glu Asn Arg
            180             185             190

Leu Pro Ser Ser Arg Trp Tyr Ser Gly Ser Gly Ile Tyr Arg Asp Val
            195             200             205

Thr Leu Thr Val Thr Asp Gly Val His Val Gly Asn Asn Gly Val Ala
    210             215             220

Ile Lys Thr Pro Ser Leu Ala Thr Gln Asn Gly Gly Asp Val Thr Met
225             230             235             240

Asn Leu Thr Thr Lys Val Ala Asn Asp Thr Glu Ala Ala Ala Asn Ile
            245             250             255

Thr Leu Lys Gln Thr Val Phe Pro Lys Gly Gly Lys Thr Asp Ala Ala
            260             265             270

Ile Gly Thr Val Thr Thr Ala Ser Lys Ser Ile Ala Ala Gly Ala Ser
            275             280             285

Ala Asp Val Thr Ser Thr Ile Thr Ala Ala Ser Pro Lys Leu Trp Ser
```

290                    295                    300

Ile Lys Asn Pro Asn Leu Tyr Thr Val Arg Thr Glu Val Leu Asn Gly
305                    310              315                320

Gly Lys Val Leu Asp Thr Tyr Asp Thr Glu Tyr Gly Phe Arg Trp Thr
                   325              330              335

Gly Phe Asp Ala Thr Ser Gly Phe Ser Leu Asn Gly Glu Lys Val Lys
              340              345              350

Leu Lys Gly Val Ser Met His His Asp Gln Gly Ser Leu Gly Ala Val
              355              360              365

Ala Asn Arg Arg Ala Ile Glu Arg Gln Val Glu Ile Leu Gln Lys Met
              370              375              380

Gly Val Asn Ser Ile Arg Thr Thr His Asn Pro Ala Ala Lys Ala Leu
385              390              395              400

Ile Asp Val Cys Asn Glu Lys Gly Val Leu Val Val Glu Glu Val Phe
              405              410              415

Asp Met Trp Asn Arg Ser Lys Asn Gly Asn Thr Glu Asp Tyr Gly Lys
              420              425              430

Trp Phe Gly Gln Ala Ile Ala Gly Asp Asn Ala Val Leu Gly Gly Asp
              435              440              445

Lys Asp Glu Thr Trp Ala Lys Phe Asp Leu Thr Ser Thr Ile Asn Arg
              450              455              460

Asp Arg Asn Ala Pro Ser Val Ile Met Trp Ser Leu Gly Asn Glu Met
465              470              475              480

Met Glu Gly Ile Ser Gly Ser Val Ser Gly Phe Pro Ala Thr Ser Ala
              485              490              495

Lys Leu Val Ala Trp Thr Lys Ala Ala Asp Ser Thr Arg Pro Met Thr
              500              505              510

Tyr Gly Asp Asn Lys Ile Lys Ala Asn Trp Asn Glu Ser Asn Thr Met
              515              520              525

Gly Asp Asn Leu Thr Ala Asn Gly Gly Val Val Gly Thr Asn Tyr Ser
              530              535              540

27

```
Asp Gly Ala Asn Tyr Asp Lys Ile Arg Thr Thr His Pro Ser Trp Ala
545                 550             555                 560

Ile Tyr Gly Ser Glu Thr Ala Ser Ala Ile Asn Ser Arg Gly Ile Tyr
                565             570                 575

Asn Arg Thr Thr Gly Gly Ala Gln Ser Ser Asp Lys Gln Leu Thr Ser
                580             585                 590

Tyr Asp Asn Ser Ala Val Gly Trp Gly Ala Val Ala Ser Ser Ala Trp
                595             600                 605

Tyr Asp Val Val Gln Arg Asp Phe Val Ala Gly Thr Tyr Val Trp Thr
                610             615                 620

Gly Phe Asp Tyr Leu Gly Glu Pro Thr Pro Trp Asn Gly Thr Gly Ser
625                 630             635                 640

Gly Ala Val Gly Ser Leu Ala Val Ala Glu Glu Leu Val Leu Arg His
                645             650                 655

Arg Arg His Arg Arg Leu Pro Glu Asp Thr Tyr Tyr Phe Tyr Gln Ser
                660             665             670

Gln Trp Asn Asp Asp Val His Thr Leu His Ile Leu Pro Ala Trp Asn
                675             680                 685

Glu Asn Val Val Ala Lys Gly Ser Gly Asn Asn Val Pro Val Val Val
                690             695             700

Tyr Thr Asp Ala Ala Lys Val Lys Leu Tyr Phe Thr Pro Lys Gly Ser
705                 710             715                 720

Thr Glu Lys Arg Leu Ile Gly Glu Lys Ser Phe Thr Lys Lys Thr Thr
                725             730                 735

Ala Ala Gly Tyr Thr Tyr Gln Val Tyr Glu Gly Ser Asp Lys Asp Ser
                740             745             750

Thr Ala His Lys Asn Met Tyr Leu Thr Trp Asn Val Pro Trp Ala Glu
                755             760                 765

Gly Thr Ile Ser Ala Glu Ala Tyr Asp Glu Asn Asn Arg Leu Ile Pro
                770             775             780

Glu Gly Ser Thr Glu Gly Asn Ala Ser Val Thr Thr Thr Gly Lys Ala
785                 790             795                 800
```

Ala Lys Leu Lys Ala Asp Ala Asp Arg Lys Thr Ile Thr Ala Asp Gly
805 810 815

Lys Asp Leu Ser Tyr Ile Glu Val Asp Val Thr Asp Ala Asn Gly His
820 825 830

Ile Val Pro Asp Ala Ala Asn Arg Val Thr Phe Asp Val Lys Gly Ala
835 840 845

Gly Lys Leu Val Gly Val Asp Asn Gly Ser Ser Pro Asp His Asp Ser
850 855 860

Tyr Gln Ala Asp Asn Arg Lys Ala Phe Ser Gly Lys Val Leu Ala Ile
865 870 875 880

Val Gln Ser Thr Lys Glu Ala Gly Glu Ile Thr Val Thr Ala Lys Ala
885 890 895

Asp Gly Leu Gln Ser Ser Thr Val Lys Ile Ala Thr Thr Ala Val Pro
900 905 910

Gly Thr Ser Thr Glu Lys Thr Val Arg Ser Phe Tyr Tyr Ser Arg Asn
915 920 925

Tyr Tyr Val Lys Thr Gly Asn Lys Pro Ile Leu Pro Ser Asp Val Glu
930 935 940

Val Arg Tyr Ser Asp Gly Thr Ser Asp Arg Gln Asn Val Thr Trp Asp
945 950 955 960

Ala Val Ser Asp Asp Gln Ile Ala Lys Ala Gly Ser Phe Ser Val Ala
965 970 975

Gly Thr Val Ala Gly Gln Lys Ile Ser Val Arg Val Thr Met Ile Asp
980 985 990

Glu Ile Gly Ala Leu Leu Asn Tyr Ser Ala Ser Thr Pro Val Gly Thr
995 1000 1005

Pro Ala Val Leu Pro Gly Ser Arg Pro Ala Val Leu Pro Asp Gly
1010 1015 1020

Thr Val Thr Ser Ala Asn Phe Ala Val His Trp Thr Lys Pro Ala
1025 1030 1035

Asp Thr Val Tyr Asn Thr Ala Gly Thr Val Lys Val Pro Gly Thr
1040 1045 1050

```
Ala Thr  Val Phe Gly Lys Glu  Phe Lys Val Thr Ala  Thr Ile Arg
    1055             1060              1065

Val Gln  Arg Ser Gln Val Thr  Ile Gly Ser Ser Val  Ser Gly Asn
    1070             1075              1080

Ala Leu  Arg Leu Thr Gln Asn  Ile Pro Ala Asp Lys  Gln Ser Asp
    1085             1090              1095

Thr Leu  Asp Ala Ile Lys Asp  Gly Ser Thr Thr Val  Asp Ala Asn
    1100             1105              1110

Thr Gly  Gly Gly Ala Asn Pro  Ser Ala Trp Thr Asn  Trp Ala Tyr
    1115             1120              1125

Ser Lys  Ala Gly His Asn Thr  Ala Glu Ile Thr Phe  Glu Tyr Ala
    1130             1135              1140

Thr Glu  Gln Gln Leu Gly Gln  Ile Val Met Tyr Phe  Phe Arg Asp
    1145             1150              1155

Ser Asn  Ala Val Arg Phe Pro  Asp Ala Gly Lys Thr  Lys Ile Gln
    1160             1165              1170

Ile Ser  Ala Asp Gly Lys Asn  Trp Thr Asp Leu Ala  Ala Thr Glu
    1175             1180              1185

Thr Ile  Ala Ala Gln Glu Ser  Ser Asp Arg Val Lys  Pro Tyr Thr
    1190             1195              1200

Tyr Asp  Phe Ala Pro Val Gly  Ala Thr Phe Val Lys  Val Thr Val
    1205             1210              1215

Thr Asn  Ala Asp Thr Thr Thr  Pro Ser Gly Val Val  Cys Ala Gly
    1220             1225              1230

Leu Thr  Glu Ile Glu Leu Lys  Thr Ala Thr Ser Lys  Phe Val Thr
    1235             1240              1245

Asn Thr  Ser Ala Ala Leu Ser  Ser Leu Thr Val Asn  Gly Thr Lys
    1250             1255              1260

Val Ser  Asp Ser Val Leu Ala  Ala Gly Ser Tyr Asn  Thr Pro Ala
    1265             1270              1275

Ile Ile  Ala Asp Val Lys Ala  Glu Gly Glu Gly Asn  Ala Ser Val
```

30

1280      1285      1290

Thr Val Leu Pro Ala His Asp Asn Val Ile Arg Val Ile Thr Glu
1295      1300      1305

Ser Glu Asp His Val Thr Arg Lys Thr Phe Thr Ile Asn Leu Gly
1310      1315      1320

Thr Glu Gln Glu Phe Pro Ala Asp Ser Asp Glu Arg Asp Tyr Pro
1325      1330      1335

Ala Ala Asp Met Thr Val Thr Val Gly Ser Glu Gln Thr Ser Gly
1340      1345      1350

Thr Ala Thr Glu Gly Pro Lys Lys Phe Ala Val Asp Gly Asn Thr
1355      1360      1365

Ser Thr Tyr Trp His Ser Asn Trp Thr Pro Thr Thr Val Asn Asp
1370      1375      1380

Leu Trp Ile Ala Phe Glu Leu Gln Lys Pro Thr Lys Leu Asp Ala
1385      1390      1395

Leu Arg Tyr Leu Pro Arg Pro Ala Gly Ser Lys Asn Gly Ser Val
1400      1405      1410

Thr Glu Tyr Lys Val Gln Val Ser Asp Asp Gly Thr Asn Trp Thr
1415      1420      1425

Asp Ala Gly Ser Gly Thr Trp Thr Thr Asp Tyr Gly Trp Lys Leu
1430      1435      1440

Ala Glu Phe Asn Gln Pro Val Thr Thr Lys His Val Arg Leu Lys
1445      1450      1455

Ala Val His Thr Tyr Ala Asp Ser Gly Asn Asp Lys Phe Met Ser
1460      1465      1470

Ala Ser Glu Ile Arg Leu Arg Lys Ala Val Asp Thr Thr Asp Ile
1475      1480      1485

Ser Gly Ala Thr Val Thr Val Pro Ala Lys Leu Thr Val Asp Arg
1490      1495      1500

Val Asp Ala Asp His Pro Ala Thr Phe Ala Thr Lys Asp Val Thr
1505      1510      1515

```
Val Thr  Leu Gly Asp Ala Thr  Leu Arg Tyr Gly Val  Asp Tyr Leu
    1520             1525             1530

Leu Asp  Tyr Ala Gly Asn Thr  Ala Val Gly Lys Ala  Thr Val Thr
    1535             1540             1545

Val Arg  Gly Ile Asp Lys Tyr  Ser Gly Thr Val Ala  Lys Thr Phe
    1550             1555             1560

Thr Ile  Glu Leu Lys Asn Ala  Pro Ala Pro Glu Pro  Thr Leu Thr
    1565             1570             1575

Ser Val  Ser Val Lys Thr Lys  Pro Ser Lys Leu Thr  Tyr Val Val
    1580             1585             1590

Gly Asp  Ala Phe Asp Pro Ala  Gly Leu Val Leu Gln  His Asp Arg
    1595             1600             1605

Gln Ala  Asp Arg Pro Pro Gln  Pro Leu Val Gly Glu  Gln Ala Asp
    1610             1615             1620

Glu Arg  Gly Leu Thr Cys Gly  Thr Arg Cys Asp Arg  Val Glu Gln
    1625             1630             1635

Leu Arg  Lys His Glu Asn Arg  Glu Ala His Arg Thr  Gly Leu Asp
    1640             1645             1650

His Leu  Glu Phe Val Gly Ala  Ala Asp Gly Ala Val  Gly Glu Gln
    1655             1660             1665

Ala Thr  Phe Lys Val His Val  His Ala Asp Gln Gly  Asp Gly Arg
    1670             1675             1680

His Asp  Asp Ala Asp Glu Arg  Asp Ile Asp Pro His  Val Pro Val
    1685             1690             1695

Asp His  Ala Val Gly Glu Leu  Ala Arg Ala Ala Cys  His His Val
    1700             1705             1710

Ile Gly  Leu Arg Val Asp Thr  His Arg Leu Lys Ala  Ser Gly Phe
    1715             1720             1725

Gln Ile  Pro Ala Asp Asp Met  Ala Glu Ile Asp Arg  Ile Thr Gly
    1730             1735             1740

Phe His  Arg Phe Glu Arg His  Val Gly
    1745             1750
```